# EUROPEAN PATENT APPLICATION

(11) **EP 1 505 517 A1**
(43) Date of publication of application: **09.02.2005**
(21) Application number: 03723335.0
(22) Date of filing: 12.05.2003
(51) Int. Cl.: G06F 17/30, C12M 1/00, C12N 15/00

(54) **MUTANT SEQUENCE ANALYZER**

(30) Priority: 10.05.2002 JP 2002136164
(71) Applicant: Celestar Lexico-Sciences, Inc., Chiba-shi, Chiba 261-8501 (JP)
(72) Inventor: IMAI, Kensaku Celestar Lexico-Sciences, Inc., Chiba-shi, Chiba 261-8501 (JP); HORAI, Naoyuki Celestar Lexico-Sciences, Inc., Chiba-shi, Chiba 261-8501 (JP); DOI, Hirofumi Celestar Lexico-Sciences, Inc., Chiba-shi, Chiba 261-8501 (JP)
(74) Representative: HOFFMANN - EITLE
(86) International application number: PCT/JP2003/005893
(87) International publication number: WO 2003/096223

(57) **Abstract**

All DNA's of a mutant of a sequencing target organic species (e.g., a resistant bacterium, a tumor cell, or a virus) are extracted, and shotgun libraries are generated for the DNA's. DNA fragments are sequentially fetched from these libraries, and each fragment is subjected to sequencing using an automated DNA sequencer. Fragment sequence information such as base sequences of the respective DNA fragments of the mutant output from the sequencer is stored in a fragment sequence database.

## Description

### TECHNICAL FIELD

The present invention relates to a mutant sequence analyzing apparatus, a mutant sequence analyzing method, a program, and a recording medium. More specifically, the present invention relates to a mutant sequence analyzing apparatus, a mutant sequence analyzing method, a program, and a recording medium capable of efficiently executing assembling and mutant part identification for a DNA sequence of a mutant.

The present invention also relates to a sequence information processing apparatus, a sequence information processing method, a program, and a recording medium. More specifically, the present invention relates to a sequence information processing apparatus, a sequence information processing method, a program, and a recording medium for processing a partial sequence of a DNA sequence or an amino acid sequence.

### BACKGROUND ART

(I) Presently, drug resistant bacteria have emerged among various bacteria such as penicillin-resistant Streptococcus pneumoniae (PRSP), cephamycin-resistant Streptococcus pneumoniae, methicillin-resistant Staphylococcus aureus (MRSA). The emergence of the drug resistant bacteria causes serious problems in clinical field. In addition, the emergence of bacteria which has resistance against several antibiotics such as multiple drug resistant Salmonella, vancomycin-resistant enterococci (VRE), and multiple drug resistant tubercle bacillus causes serious problems. A drastic solution to the resistant bacteria has been, therefore, demanded. Under these circumstances, for obtaining important information used in clarification of a resistance obtaining mechanism, confirmation of characters of resistant bacteria, development of resistant bacteria treatments, and the like, a gene analysis of resistant bacteria has been conducted.

Furthermore, it has been clear that not only such resistant bacteria but also acquisition of the drug resistance due to base sequence mutation of AIDS virus, cancer cells and the like are involved by a multi drug resistant gene (MDRI) and the like. An analysis of this gene mutation has been conducted, accordingly.

A conventional gene mutation analysis requires, first of all, decoding base sequence information on a DNA of a resistant bacteria or the like. As a sequence determination scheme, random shotgun sequencing is widely employed. Procedures from determination of a base sequence of a genome to addition of annotation according to this scheme will now be explained (see Toshihisa TAKAGI, et al. *Genome Information Biology*, pages 2 to 18, 2000, Nakayama Shoten Co., Ltd.).

### [1. Generation of Sequencing Fragment]

All DNA's of a sequencing target organic species are first extracted, and shotgun libraries for the DNA's are generated. Fragments are sequentially fetched from these libraries, and each of the fragments is subjected to sequencing using an automated DNA sequencer. As sequencing methods, a method for sequencing each fragment from both ends of the fragment and a method for sequencing the fragment only from one end of the fragment are known.

### [2. Management of Base Calling and Sequence Quality]

Base sequences of the respective fragments are output from the sequencer. Before the output, base calling and sequence quality determination are performed. Software having functions of the base calling and the sequence quality determination is often installed in the sequencer. A software product for base calling from waveform data, that for a base-called sequence evaluation, and the like are also provided.

### [3. Elimination of Contaminations from a heterogeneous DNA, Vector DNA to be used, and the like]

Using a homology analysis, contaminations are eliminated from a cloning vector, a sequence vector, or the like.

### [4. Detection of Overlap among Fragments]

At an assembling stage, an overlap among the fragments is detected throughout the entire libraries, and assembling results are stored, respectively.

### [5. Clustering and Generation of Contigs]

Thereafter, clustering and generation of contigs are executed based on the assembling results. At an initial stage of the assembling stage, the number of contigs increases in proportion with an increase in the number of fragments, and a size of each contig is enlarged, accordingly. However, when a redundancy (a degree of the overlap among the fragments) exceeds about 1.0, a sum of contig lengths has a slow increase. When the redundancy exceeds about 5.0, the sum of contig lengths hardly increases. At this stage, even if new fragments are fetched from the libraries, gaps between the contigs cannot be satisfactorily filled in. On the other hand, most regions of relatively long contigs are covered by the number of fragments that indicate an average redundancy, and consensus sequences are stabilized. Therefore, a comparison analysis of the contigs, an identification of an ORF (open reading frame) within each contig, an identification of a transcription unit, and the like are executable at this stage. Almost accurate consensus sequences are determined for these contigs except for both ends thereof and partial regions thereof in each of which the overlap is small.

### [6. Primer Designing and Primer Walking in Both End Regions of Contig]

The stage at which the number of gaps does not increase greatly even when the number of fragments increases moves to a gap filling stage. To fill in the gaps, primer walking sequencing is performed. Specifically, primers are designed so as to be directed from both end regions of each contig toward outsides of the regions, and the obtained primers are mixed up, and clones that bridge the contigs are obtained from all the DNA's by PCR (polymerase chain reaction). Both ends of each clone are sequenced, thereby filling in the gaps. This method allows each contig to be enlarged by as much as one fragment whenever the primer walking sequencing is performed. The obtained base sequences are re-assembled, thereby filling in the gaps. If a gap is short, the gap is sometimes closed only by one primer walking sequencing. For the gaps that are not filled in, a reassembling operation is further carried out and repeated until all the gaps are filled in. Nevertheless, gaps that are not filled in finally are also present in a DNA that is present separately in a plurality of chromosomes. Therefore, a processing for generating a cyclic form is required.

### [7. Annotation]

Using a coding region prediction program or the like, an ORF is identified first. Each of the coding region is translated to amino acids, and an identification of functions based on an amino acid sequence is performed, as well. Further, annotation is performed based on comparison with genomes of other organic species, thus identifying a so-called orthologue relationship. In parallel to the processing, ORF maps for all genomes are generated. For those gene functions of which have been determined so far, maps separated according to structure and function are generated, respectively.

However, it requires much time, labor, and the like to perform the assembling processing based on this method. Accordingly, the conventional analysis has a basic disadvantage related to a system structure. That is, pieces of base sequence information on mutant DNA's of massive and diversified resistant bacteria cannot be promptly analyzed.

A content of this disadvantage will be explained more specifically.

According to the conventional analysis, after determining whole genome sequences of a mutant, a mutant site is extracted by a comparison with a DNA sequence of an original wild strain or the like before mutation. Accordingly, it is necessary to make an adjustment of the libraries, generation of fragments, and the like for the whole genomes. Accordingly, a heavy burden of a wet experiment is disadvantageously cast on the conventional analysis.

Furthermore, after executing an alignment of a vast number of fragment sequences on a calculator for assembling, it is necessary to edit and check the base sequences. Accordingly, a calculator-side operational burden is also disadvantageously cast on the conventional analysis.

The overlap (redundancy) among the fragment sequences is required when the assembling is to be performed. Accordingly, accurate assembling cannot be expected on small-overlap parts, i.e., low redundancy parts or low fragment sequence quality parts of the fragment sequence.

Moreover, to connect the low-redundancy parts of the fragment sequence or contigs, it is necessary to design PCR primers directed toward outsides of the both ends of the contigs using a region which is close to the both ends of each contig and in which the sequence has a specificity as high as possible. Whenever the PCR primer is designed, it is required to do so manually using dedicated software or the like. Accordingly, the conventional analysis has a disadvantage in that automation of operation and improvement of efficiency thereof cannot be realized.

Furthermore, if conventional software such as a genome viewer that displays assembling results is used, parts that cannot be displayed on one screen are displayed by scrolling or information is displayed by vertically dividing a display. However, for a long base sequence, it is disadvantageously difficult to intuitively grasp a redundancy and a mutant part of the sequence.

As can be seen, the conventional system and the like are confronted with many disadvantages. Accordingly, the conventional system and the like are inferior in convenience and utilization efficiency for both a user and an administrator of the system and the like.

Namely, genome sequencing is conventionally performed for a variety of species. However, the conventional analysis technique is an ordinary technique mainly for novel (or totally unknown) genome sequencing, and intended to eliminate contradictions while referring to assembling based on incomplete maps, and to thereby generate contigs. Possible genome sequencing frequently performed in the future, by contrast, is executed based on assumption that known genome sequences having high homologies are comparison targets and is intended to analyze such genome sequences repeatedly in large amounts. In addition, sequencing that can determine the whole genomes and that covers the whole genomes will not be always performed. The present invention provides one of practical solutions under these circumstances.

It is, therefore, an object of the present invention to provide a mutant sequence analyzing apparatus, a mutant sequence analyzing method, a program, and a recording medium capable of efficiently checking a mutant status and the like of a DNA sequence or the like of a mutant at real time.

(II) Meanwhile, information widely used in the bioinformatics field includes base and amino acid sequence data. Analysis of diversified life phenomena has been performed at present by conducting a homology search, a motif search, or the like based on the sequence data stored in various databases. An analysis scheme that uses partial sequences of the sequence has been recently studied in the process of the life phenomenon analyses using the sequence information.

The invention disclosed in Japanese Patent Application Laid-Open No. H10-45795, for example, relates to a protein database system and a protein function and functional site estimating method for estimating a function and a functional site of a function-unknown protein or a functional site of a function-known but functional site-unknown protein using partial sequences. Namely, the Japanese Patent Application Laid-Open No. H10-45795 discloses the invention that displays information on functions and the like related to oligopeptides which information is a part of amino acid sequence information on a specific protein, and a protein that contains the oligopeptides, an expression frequency of the oligopeptides, and the like.

The invention disclosed in Japanese Patent Application No. 2000-72485 relates to a protein interaction predicting method using partial sequences and a recording medium having the prediction program recorded therein. Namely, the Japanese Patent Application No. 2000-72485 discloses the invention that predicts an interaction between proteins using information on an oligopeptide sequence which is a part of an amino acid sequence of a specific protein, and information on an alignment result of the specific protein relative to the other protein having the oligopeptide sequence information, an expression frequency of the oligopeptides, and the like.

These conventional techniques have, however, the following disadvantages. Neither of the conventional techniques is intended to comprehensively collect information on partial sequences of all oligopeptides and the like, and displays the collected information in a table so as to facilitate understanding. In other words, neither of the conventional techniques arranges, stores, searches, and manages all pieces of information on the partial sequences while making them correspond to the partial sequences.

According to the inventions disclosed in the Japanese Patent Application Laid-Open No. H10-45795 and the Japanese Patent Application No. 2000-72485, for example, when large amounts of information on the oligopeptide sequence data are to be displayed on an output device, a user is unable to easily make a setting as to which information is displayed how much, and to store the setting, and to search the setting later for reuse.

That is, according to the conventional art, the user is unable to easily make the setting as to which information is chosen to be displayed, store the setting, and search the setting later for reuse. Moreover, neither of the conventional oligopeptide analysis schemes automatically enlarges a search range up to mutants of the oligopeptides and searches sequence information.

As can be seen, the partial sequence analysis, particularly the analysis of oligopeptides that are partial sequences of the amino acid sequence, is remarkably effective since it is known that the analysis can be used when estimating the functions, interaction, and the like of a protein. The conventional analysis schemes have, however, these several disadvantages, and are inferior in convenience and utilization efficiency for both a user and an administrator of the sequence information display apparatus.

The above-described disadvantages which the conventional art are confronted with and which the present invention is to solve, should be overcome not only for the system that displays sequence information on oligopeptides but also for all systems that display partial sequences of sequence information on all kinds.

It is, therefore, an object of the present invention to provide a sequence information processing apparatus, a sequence information processing method, a program, and a recording medium which enable a user to easily make a setting as to which sequence information and which related information to the sequence information is to be chosen so as to display the information on an output device, to store the setting, and to search the setting later so that the user can reuse the setting.

### DISCLOSURE OF THE INVENTION

(I) It is an object of the present invention to efficiently obtain a consensus sequence of a mutant genome. Namely, according to the present invention, differently from the conventionally used scheme of determining the genome sequence of the mutant by assembling respective fragments of the mutant genome, each fragment is assigned a position on a sequence of a normal type (e.g., a wild strain), a local consensus sequence is determined from locally overlapping fragments, and the local consensus sequences are connected, thereby determining a genome sequence of the entire mutant genome.

As explained, according to the conventional method using assembling, it is necessary to perform a sequencing operation for a vast number of fragments so as to generate a complete genome sequence. In addition, even if a high redundancy is present over the entire genome, it is difficult to fill in gaps and also difficult to determine an order of contigs, a contig direction, and the like.

According to the present invention, a position on the sequence of the normal type is assigned to each fragment of the mutant, a local consensus sequence is determined based on the position-assigned fragments, and these local consensus sequences are connected, thereby determining a genome sequence of all the mutant genomes. Therefore, even if the number of fragments is small, contigs can be easily generated, and specification of gaps (specification of consecutive contigs, specification of a direction of each contig, specification of a distance, and the like) can be easily performed. Further, according to the present invention, the assembling operation is not required. It is, therefore, possible to predict the genome sequence of the mutant using pieces of fragment data analyzed so far at an arbitrary timing during a fragment sequencing operation. Besides, because of lack of the assembling operation, extraction calculation of overall statistic information can be easily made.

Moreover, according to the present invention, an experimentalist (a user) specifies a region in which the experimentalist (user) is to specify the genome sequence (a region of interest) in the sequence of the normal type. It is thereby possible to perform the fragment sequencing operation specific to the region of interest, and check a sequence status of the region of interest at real time.

Additionally, according to the present invention, a low redundancy region or the like of the sequenced fragment is detected so as to complete an accurate consensus sequence. Namely, mutation can be detected at locations at each of which the local consensus sequence of the mutant genome can be determined. However, locations at each of which the local consensus sequence cannot be determined (e.g., a low redundancy part of the fragment, and a gap part) can be recognized as additional sequencing target locations. In addition, a PCR primer sequence used for the sequencing can be determined.

To attain such an object, a mutant sequence analyzing apparatus, a mutant sequence analyzing method, and a program according to the present invention comprise (include): a fragment sequence storage unit that stores (a fragment sequence storage step of storing) fragment sequence information on a base sequence of each DNA fragment of a mutant (which information may include, for example, DNA sequence information on each fragment of the mutant output from a DNA sequencer, waveform information on the DNA sequence information, information on a sequence quality, and a DNA sequence of a vector used in a sequencing experiment); a genome information storage unit that stores (a genome information storage step of storing) original DNA sequence information before mutation (which information may include, for example, amino acid sequence information, information on organic species, and information on functions as well as the DNA sequence); a region-of-interest selection unit that selects (a region-of-interest selection step of selecting) a region of interest composed by a sequence region of at least a part of the original DNA sequence information stored in the genome information storage unit (at the genome information storage step); a homology search unit that determines (a homology search step of determining) a similarity between the region of interest selected by the region-of-interest selection unit (at the region-of-interest selection step) and the fragment sequence information stored in the fragment sequence storage unit (at the fragment sequence storage step); a fragment adoption determination unit that determines (a fragment adoption determination step of determining) whether to adopt the fragment sequence information as the fragment sequence information used for sequencing the mutation, according to the similarity determined by the homology search unit (at the homology search step); an alignment processing unit that makes (an alignment processing step of making) an alignment between the fragment sequence information determined to be adopted by the fragment adoption determination unit (at the fragment adoption determination step) and the region of interest; and a consensus sequence determination unit that determines (a consensus sequence determination step of determining) a consensus sequence of the mutant based on an alignment result of the alignment processing unit (the alignment processing step).

According to the apparatus, the method, and the program, fragment sequence information on a base sequence of each DNA fragment of a mutant is stored, original DNA sequence information before mutation is stored, a region of interest composed by a sequence region of at least a part of the stored original DNA sequence information stored is selected, a similarity between the selected region of interest and the stored fragment sequence information is determined, it is determined whether to adopt the fragment sequence information as the fragment sequence information used for sequencing the mutation according to the determined similarity, an alignment between the fragment sequence information determined to be adopted and the region of interest is made, and a consensus sequence of the mutant is determined based on an alignment result. Therefore, by specifying the region of interest predicted to undergo mutation, and generating the consensus sequence from the fragments of the mutant assigned to the region of interest, the DNA sequence of the mutant part can be efficiently determined.

Namely, according to the conventional art, it is necessary to perform the operation for determining the sequences of all genomes of the mutant. Accordingly, it is necessary to perform adjustment of libraries, generation of fragments, and the like for all the genomes, thereby casting heavy burden of the wet experiment on the conventional analysis. In addition, after executing an alignment of a vast number of fragment sequences on the calculator for assembling, it is necessary to edit and check the base sequences. Accordingly, a calculator-side operational burden is also cast on the conventional analysis. According to the apparatus of the present invention, it is unnecessary to determine the sequences of all genomes, and it suffices only to experiment and calculate only the region of interest. Therefore, various resources can be efficiently utilized, and the DNA sequence of the mutant can be promptly and accurately determined.

Furthermore, the conventional assembling requires overlap (redundancy) among the fragment sequences. Accordingly, accurate assembling cannot be expected on small-overlap parts, i.e., low redundancy parts or low fragment sequence quality parts of the fragment sequence. According to the apparatus of the present invention, the consensus sequence is generated using the fragments assigned to the original DNA sequence information before mutation even for a part in which it is difficult to perform assembling. Therefore, the base sequence can be identified or estimated with high accuracy.

The mutant sequence analyzing apparatus, the mutant sequence analyzing method, and the program according to the next invention, based on the mutant sequence analyzing apparatus, the mutant sequence analyzing method, and the program explained above, further comprise (include) a mutation information determination unit that determines (a mutation information determination step of determining) mutation information by comparing the consensus sequence determined by the consensus sequence determination unit (at the consensus sequence determination step) with the region of interest.

According to the apparatus, the method, and the program, mutation information is determined by comparing the determined consensus sequence with the region of interest. Therefore, the sequence of the mutant part of the mutant can be automatically determined.

The mutation information may include, for example, positions of mutations, the mutated bases, a redundancy at the positions of mutations, and a sequence quality at the position of mutations (e.g., a probability of base calling from waveform data and whether both strands are sequenced). In addition, mutation information common to a plurality of variants may be determined. In addition, a plurality of gene regions having mutation correlation may be extracted.

The mutant sequence analyzing apparatus, the mutant sequence analyzing method, and the program according to the next invention, based on the mutant sequence analyzing apparatus, further comprise (include) a redundancy determination unit that determines (a redundancy determination step of determining) a redundancy of the each DNA fragment relative to each base that constitutes the region of interest based on the alignment result of the alignment processing unit (the alignment processing step).

According to the apparatus, the method, and the program, a redundancy of each DNA fragment relative to each base that constitutes the region of interest is determined based on an alignment result. Therefore, the redundancy that is important information for predicting assembling accuracy and the like can be automatically determined.

The mutant sequence analyzing apparatus, the mutant sequence analyzing method, and the program according to the next invention, based on the mutant sequence analyzing apparatus, the mutant sequence analyzing method, and the program explained above, further comprise (include) a gap information determination unit that determines (a gap information determination step of determining) gap information that specifies a gap part of the each DNA fragment relative to the region of interest based on the alignment result of the alignment processing unit (the alignment processing step).

According to the apparatus, the method, and the program, gap information that specifies a gap part of each DNA fragment relative to the region of interest is determined based on an alignment result. Therefore, the part which cannot be sequenced for the DNA sequence of the mutant, that is, a base sequence part that is present in the original DNA sequence (region of interest) before mutation but that is not present in each fragment (consensus sequence) can be automatically determined.

The mutant sequence analyzing apparatus, the mutant sequence analyzing method, and the program according to the next invention, based on the mutant sequence analyzing apparatus, the mutant sequence analyzing method, and the program explained above, further comprise (include) a processing result display unit that displays (a processing result display step of displaying) a processing result including at least one of the region of interest, annotation information on the region of interest, the consensus sequence, annotation information on the consensus sequence, the redundancy, the mutation information, the gap information, and the alignment result.

According to the apparatus, the method, and the program, a processing result including at least one of the region of interest, annotation information on the region of interest, the consensus sequence, annotation information on the consensus sequence, the redundancy, the mutation information, the gap information, and the alignment result is displayed. Therefore, it is possible to accelerate discovering a new biological knowledge by the user who checks such useful information.

The mutant sequence analyzing apparatus, the mutant sequence analyzing method, and the program according to the next invention, based on the mutant sequence analyzing apparatus, the mutant sequence analyzing method, and the program explained above, are characterized in that the annotation information on the consensus sequence includes information on at least one of an ORF region of the consensus sequence, a control region, an amino acid sequence after translation, an original amino acid sequence before mutation, a function after the mutation, an original function before the mutation, and an orthologue relationship.

This shows an example of the annotation information on the consensus sequence more specifically. According to the apparatus, the method, and the program, the annotation information on the consensus sequence includes information on at least one of an ORF region of the consensus sequence, a control region, an amino acid sequence after translation (e.g., substitutional mutation, and frame shift mutation caused by insertion or deletion), a function after the mutation (e.g., a function change caused by mutation of the gene region or that of the control region, and function correlation analysis for a plurality of gene regions having mutation correlation), and an orthologue relationship (e.g., analysis of genes of different types that hold the same mutation, and strain analysis). Therefore, by searching a known genome information database such as cDNA database, searching a similar sequence or function among cells and the like of the same type or different types, and determining information on these cells and the like, it is possible to accelerate discovering a new biological knowledge by the user who checks these pieces of useful information.

The mutant sequence analyzing apparatus, the mutant sequence analyzing method, and the program according to the next invention, based on the mutant sequence analyzing apparatus, the mutant sequence analyzing method, and the program explained above, are characterized in that the processing result display unit displays the processing result on a plurality of output devices (at the processing result display step), the processing result are displayed on a plurality of output devices by dividing the processing result by as much as the number of the output devices.

This shows an example of displaying the processing result more specifically. According to the apparatus, the method, and the program, the processing result is displayed on a plurality of output devices by dividing the processing result by as much as the number of the output devices. Therefore, similarly to an instance of displaying the processing result on a large-sized display (e.g., a horizontally-long display), the processing result can be displayed to correspond to arrangement of a plurality of output devices (e.g., displayed on a virtually horizontally-long output device if a plurality of displays are arranged in a lateral direction).

Namely, according to the present invention, an analysis operation can be easily and visually performed when, for example, checking a sequencing status (e.g., a redundancy and a gap), checking a mutant insertion status (e.g., a relative position from any of various loci of the gene or the like, and the number of mutants), checking an influence of the mutation of the base sequence on amino acid translation and the like, and checking genome information on a comparison among a plurality of resistant organisms.

The mutant sequence analyzing apparatus, the mutant sequence analyzing method, and the program according to the next invention, based on the mutant sequence analyzing apparatus, the mutant sequence analyzing method, and the program explained above, further comprise (include) a primer sequence determination unit that determines (a primer sequence determination step of determining) base sequence candidates of a PCR primer used to generate the each DNA fragment that includes both of or one of a sequence region having both of or one of the redundancy determined by the redundancy determination unit (at the redundancy determination step) and a sequence quality, each of which is lower than a preset value, and a sequence region of the gap part determined by the gap information determination unit (at the gap information determination step).

According to the apparatus, the method, and the program, base sequence candidates of a PCR primer used to generate the each DNA fragment that includes both of or one of a sequence region having both of or one of the determined redundancy and the sequence quality, each of which is lower than a preset value, and a sequence region of the determined gap part are determined. Therefore, as a result of sequencing, the PCR primer used to generate fragments that cover the gap part and the small fragment overlap part can be automatically designed, and automation of operation and improvement of efficiency can be realized.

Furthermore, it is thereby possible to efficiently design the PCR primer for supporting an experiment of rechecking of the mutant detected site, an experiment using the region of interest, and the like instead of specifying the low redundancy site or the gap part.

The mutant sequence analyzing apparatus, the mutant sequence analyzing method, and the program according to the next invention, based on the mutant sequence analyzing apparatus, the mutant sequence analyzing method, and the program explained above, are characterized in that the primer sequence determination unit (the primer sequence determination step) further comprises (includes): a partial sequence extraction unit that extracts (a partial sequence extraction step of extracting) partial sequences located outside of both of or one of the sequence region having both of or one of the redundancy determined by the redundancy determination unit (at the redundancy determination step) and the sequence quality, each of which is lower than the preset value, and the sequence region of the gap part determined by the gap information determination unit (at the gap information determination step), and each having a high specificity in each of the sequences; and a primer candidate selection unit that determines (a primer candidate selection step of determining) the base sequence candidates of the PCR primer among the partial sequences extracted by the partial sequence extraction unit (at the partial sequence extraction step) according to preset selection conditions.

This shows an example of determining the primer sequence more specifically. According to the apparatus, the method, and the program, partial sequences located outside of both of or one of the sequence region having both of or one of the determined redundancy and the sequence quality, each of which is lower than the preset value, and the sequence region of the determined gap part, and each having a high specificity in each of the sequences are extracted. Therefore, it is possible to specify one site to be annealed by the PCR primer used in the primer walking. In addition, as a determination criterion of the specificity that secures single copy, a specificity criterion from two viewpoints of an ordinary (global) sense and a (local) sense that only one site is obviously present in the wild strain of a type of interest can be used.

In addition, the base sequence candidates of the PCR primer are determined among the extracted partial sequences according to preset selection conditions. Therefore, it is possible to determine the base sequence candidates of the PCR primer after eliminating PCR primers that are annealed to each other to form a dimer, a PCR primer that has partially a higher GC or AT content, a PCR primer that includes a self-complementary sequence and that forms a secondary structure by itself, a PCR primer having a melting temperature Tm that is a temperature in a state in which 50% of a double-strand DNA forms a hydrogen bond pair and remaining 50% thereof is dissociated, and that is not between an upper limit temperature and a lower limit temperature in a PCR cycle, and the like.

The mutant sequence analyzing apparatus, the mutant sequence analyzing method, and the program according to the next invention, based on the mutant sequence analyzing apparatus, the mutant sequence analyzing method, and the program explained above, are characterized in that each of the partial sequences is a base sequence having the number of bases equal to or greater than 15 and equal to or smaller than 30.

This shows an example of the partial sequence more specifically. According to the apparatus, the method, and the program, each of the partial sequences is a base sequence having the number of bases equal to or greater than 15 and equal to or smaller than 30 (preferably 20 to 25). Therefore, the PCR can be efficiently executed. Namely, if the number of bases is equal to or smaller than 14, it is highly likely that the partial sequence cannot be satisfactorily annealed to a template. If the number of bases is equal to or greater than 31, a probability of misannealing increases. Thus, by specifying an appropriate number of bases, a high quality primer sequence can be determined.

Furthermore, the present invention relates to a recording medium. The recording medium according to the present invention is characterized by recording the above-explained program.

According to this recording medium, by making a computer read and execute the program recorded in the recording medium, the program can be realized using the computer. In addition, the same advantages as those of the respective methods can be attained.

(II) Further, to attain the object, a sequence information processing apparatus, sequence information processing method, and a program according to the present invention comprise (include): a sequence information storage unit that stores (a sequence information storage step of storing) sequence information on bases or amino acids and sequence related information related to the sequence information while making the sequence information and the sequence related information correspond to each other; a target sequence selection unit that causes (a target sequence selection step of causing) a user to select a target sequence from among the sequence information stored in the sequence information storage unit (at the sequence information storage step); a population selection unit that causes (a population selection step of causing) the user to select sequence information on a population, which is a search target, from among the sequence information stored in the sequence information storage unit (at the sequence information storage step); a sequence division unit that divides (a sequence division step) the target sequence selected by the target sequence selection unit (at the target sequence selection step) into partial sequences each having a length designated by the user; a search unit that searches (a search step) the partial sequences divided by the sequence division unit (at the sequence division step) from the population; an analysis result information generation unit that generates (an analysis result information generation step of generating) analysis result information on the target sequence from search result information on the search unit (the search step); and a table output unit that outputs (a table output step of outputting) partial sequence information on the partial sequences and the analysis result information in a table form according to parameters set by the user.

According to the apparatus, the method, and the program, sequence information on bases or amino acids and sequence related information related to the sequence information is stored while making the sequence information and the sequence related information correspond to each other, a user is caused to select a target sequence from among the stored sequence information, the user is caused to select sequence information on a population, which is a search target, from among the stored sequence information, the selected target sequence is divided into partial sequences each having a length designated by the user, the divided partial sequences are searched from the population, analysis result information on the target sequence is generated from search result information, and partial sequence information on the partial sequences and the analysis result information is output in a table form according to parameters set by the user. Therefore, the user can easily make a setting as to which sequence information and which analysis information on the sequence information is to be chosen to be displayed as a processing result, store the setting, and reuse the setting later.

The sequence information processing apparatus, the sequence information processing method, and the program according to the next invention, based on the sequence information processing apparatus, the sequence information processing method, and the program explained above, further comprise (include): a mutant sequence information storage unit that stores (a mutant sequence information storage step of storing) the sequence information and mutant sequence information on mutant sequences which may possibly be generated by mutation of the sequence information while making the sequence information and the mutant sequence information correspond to each other; and a mutant sequence search unit that searches (a mutant sequence search step of searching) mutant sequence information on the partial sequences divided by the partial sequence division unit (at the partial sequence division step) from the mutant sequence information stored in the mutant sequence information storage unit (at the mutant sequence information storage step), and are characterized in that the search unit searches (a search step of searching) the mutant information on the partial sequences searched by the mutant sequence search unit, from the population (at the search step), the mutant information on the partial sequences searched by the mutant sequence search step is searched from the population.

According to the apparatus, the method, and the program, the sequence information and mutant sequence information on mutant sequences which may possibly be generated by mutation of the sequence information is stored while making the sequence information and the mutant sequence information correspond to each other, and mutant sequence information on the divided partial sequences is searched from the stored mutant sequence information, and the mutant information on the searched partial sequences searched is searched from the population. Therefore, a search range can be enlarged up to mutants of the search target partial sequences. In addition, the information can be used to analyze more useful biological information. The sequence information processing apparatus, the sequence information processing method, and the program according to the next invention, based on the sequence information processing apparatus, the sequence information processing method, and the program explained above, are characterized in that the sequence related information includes information on at least one of a name of each sequence, a name of an organism from which the each sequence is derived, a name of a corresponding protein, functions of the protein, and an address of a related database.

This shows an example of the sequence related information more specifically. According to the apparatus, the method, and the program, the sequence related information includes information on at least one of a name of each sequence, a name of an organism from which the each sequence is derived, a name of a corresponding protein, functions of the protein, and an address of a related database. Therefore, these items of information can be used as analysis information on the target sequence.

The sequence information processing apparatus, the sequence information processing method, and the program according to the next invention, based on the sequence information processing apparatus, the sequence information processing method, and the program explained above, are characterized in that the population is generated by collecting sequence information on proteins that possess specific properties.

This shows an example of the search target population more specifically. According to the apparatus, the method, and the program, the population is generated by collecting sequence information on proteins that possess specific properties. It is, therefore, possible to limit the proteins to those necessary to extract as the analysis information, and reduce search time. It is also possible to analyze the population with respect to another target sequence.

The sequence information processing apparatus, the sequence information processing method, and the program according to the next invention, based on the sequence information processing apparatus, the sequence information processing method, and the program explained above, are characterized in that the length of each of the partial sequences is 4 to 7.

This shows an example of the length of each partial sequence more specifically. According to the apparatus, the method, and the program, the length of each of the partial sequences is 4 to 7. Therefore, biologically effective data can be accumulated within operation time of an appropriate computer.

The sequence information processing apparatus, the sequence information processing method, and the program according to the next invention, based on the sequence information processing apparatus, the sequence information processing method, and the program explained above, are characterized in that the partial sequence information includes information on at least one of a position from a top of the target sequence and the partial sequences.

This shows an example of the partial sequences more specifically. According to the apparatus, the method, and the program, the partial sequence information includes information on at least one of a position from a top of the target sequence and the partial sequences. Therefore, each partial sequence and its position can be arbitrarily displayed.

The sequence information processing apparatus, the sequence information processing method, and the program according to the next invention, based on the sequence information processing apparatus, the sequence information processing method, and the program explained above, are characterized in that the analysis result information includes information on at least one of the number of extracted sequences, the mutant sequence information used in a search, a name of each of the extracted sequences, a name of an organism from which the each extracted sequence is derived, a name of a protein corresponding to the each extracted sequence, a function of the protein corresponding to the each extracted sequence, an execution result of an external program, and an address of a related database to the each extracted sequence, for each of the partial sequences.

This shows an example of the analysis result information more specifically. According to the apparatus, the method, and the program, the analysis result information includes information on at least one of the number of extracted sequences, the mutant sequence information used in a search, a name of each of the extracted sequences, a name of an organism from which the each extracted sequence is derived, a name of a protein corresponding to the each extracted sequence, a function of the protein corresponding to the each extracted sequence, an execution result of an external program, and an address of a related database to the each extracted sequence, for each of the partial sequences. Therefore, these respective items of information can be arbitrarily displayed.

The sequence information processing apparatus, the sequence information processing method, and the program according to the next invention, based on the sequence information processing apparatus, the sequence information processing method, and the program explained above, are characterized in that the parameters include information on at least one of whether each information included in the analysis result information can be output, an upper limit of the number of output sequences among the extracted sequences, whether the each information included in the analysis result information can be graphically displayed, a header format of the table, and a data description language for displaying data in the table.

This shows an example of the analysis result information more specifically. According to the apparatus, the method, and the program, the parameters include information on at least one of whether each information included in the analysis result information can be output, an upper limit of the number of output sequences among the extracted sequences, whether the each information included in the analysis result information can be graphically displayed, a header format of the table, and a data description language for displaying data in the table. Therefore, these respective pieces of information can be arbitrarily displayed.

Furthermore, the present invention relates to a recording medium. The recording medium according to the present invention is characterized by recording the above-explained program.

According to this recording medium, by making a computer read and execute the program recorded in the recording medium, the program can be realized using the computer. In addition, the same advantages as those of the respective programs can be attained.

### BRIEF DESCRIPTION OF THE DRAWINGS

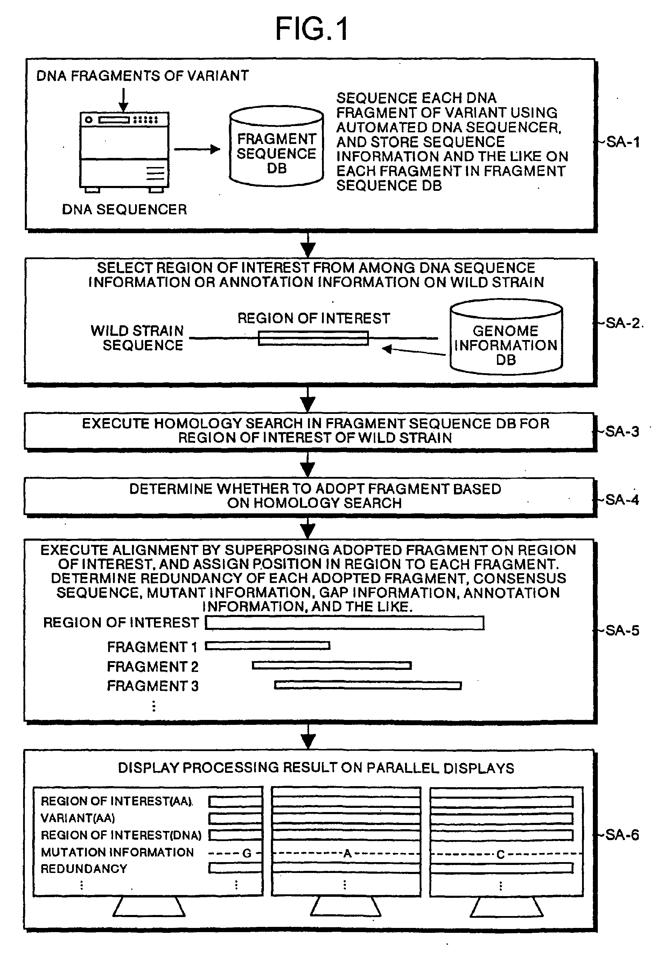

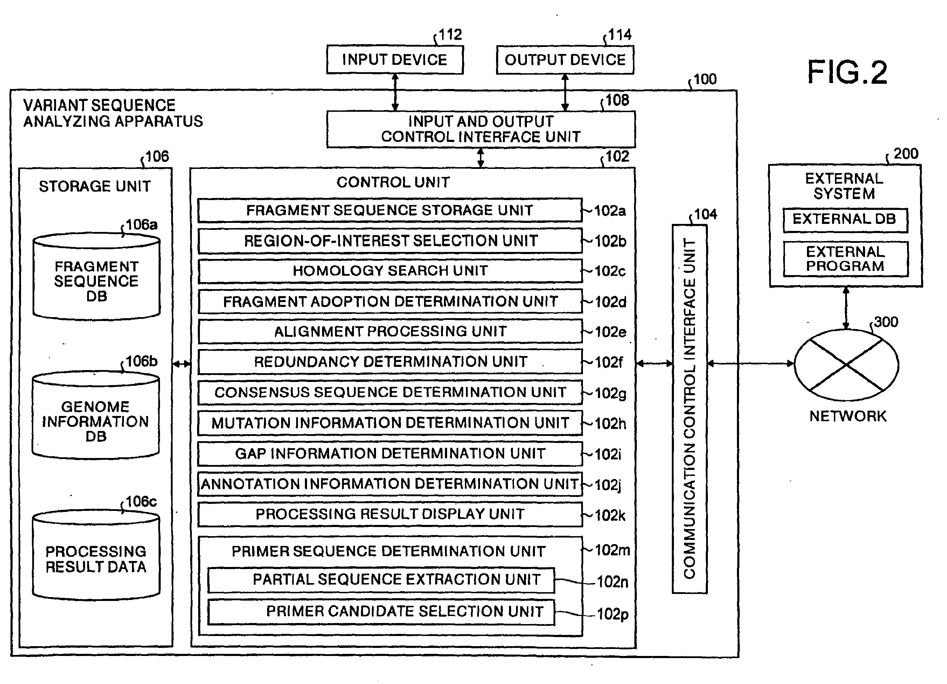

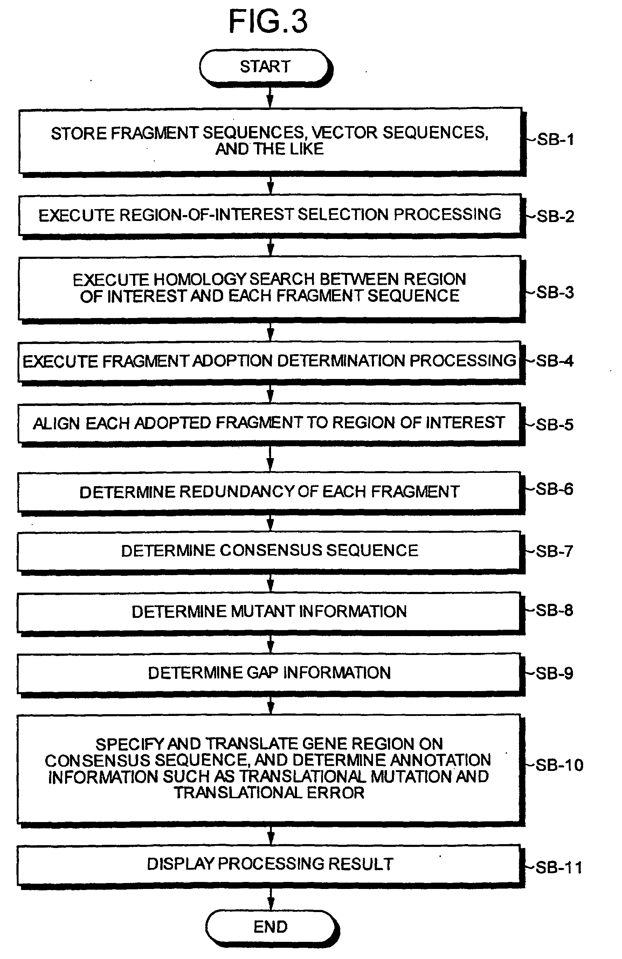

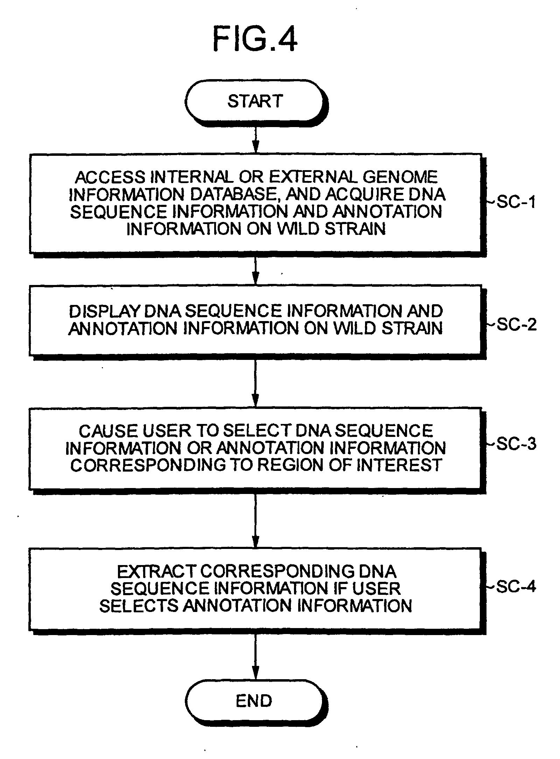

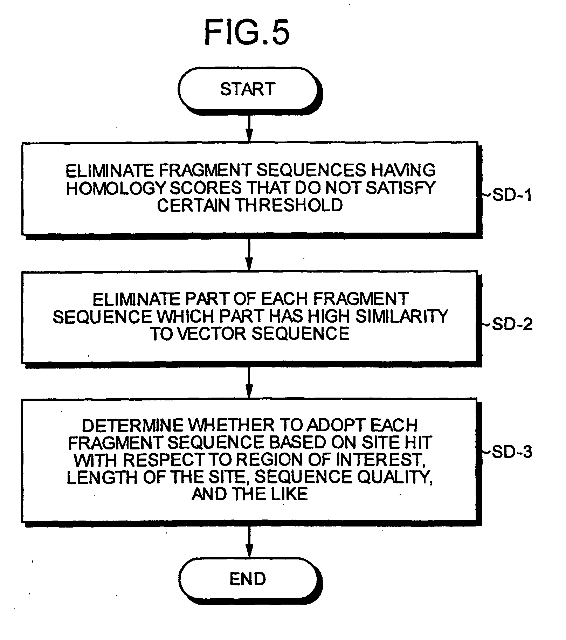

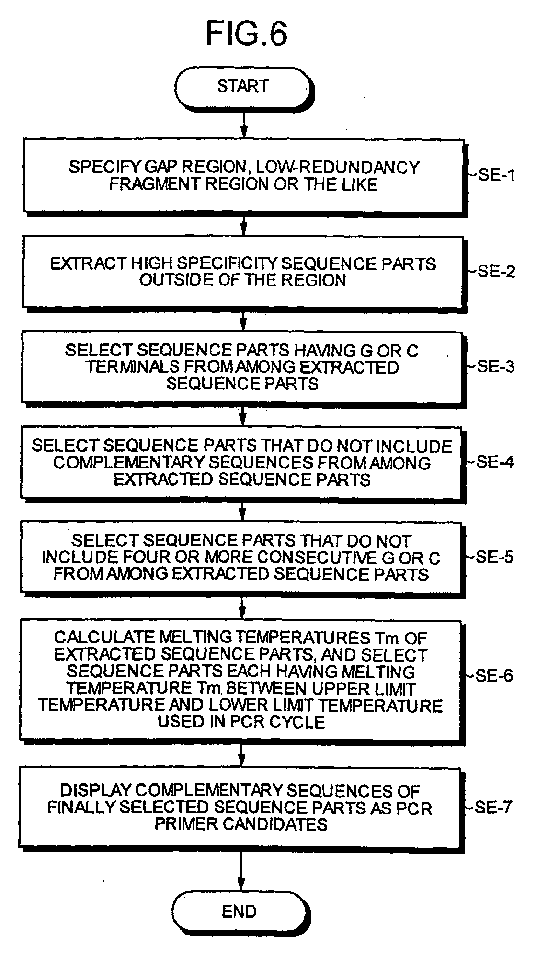

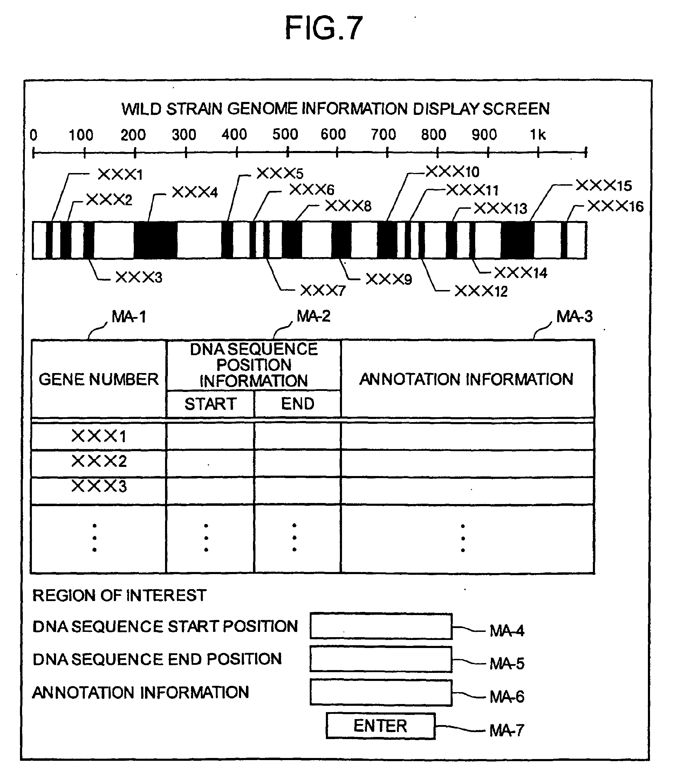

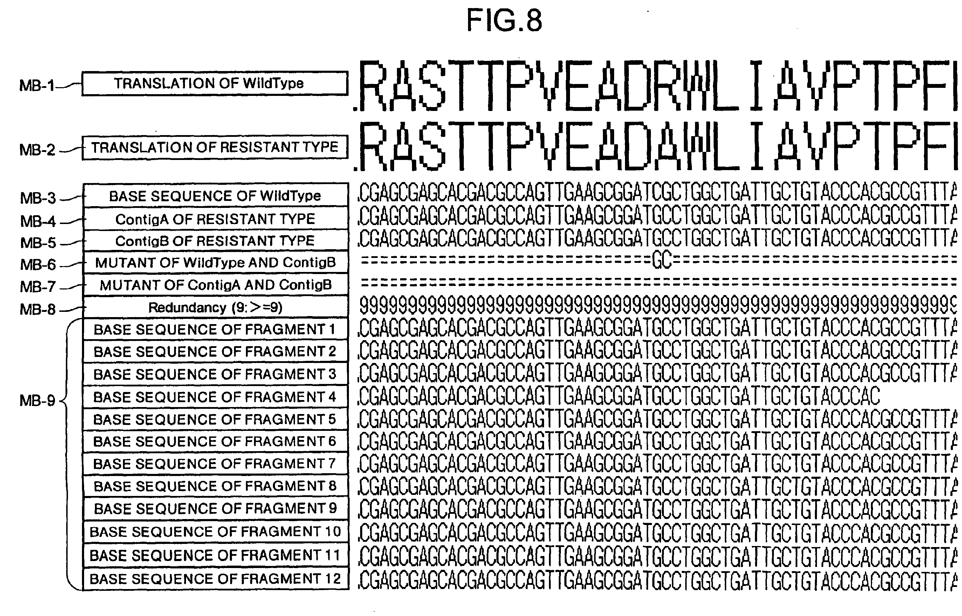

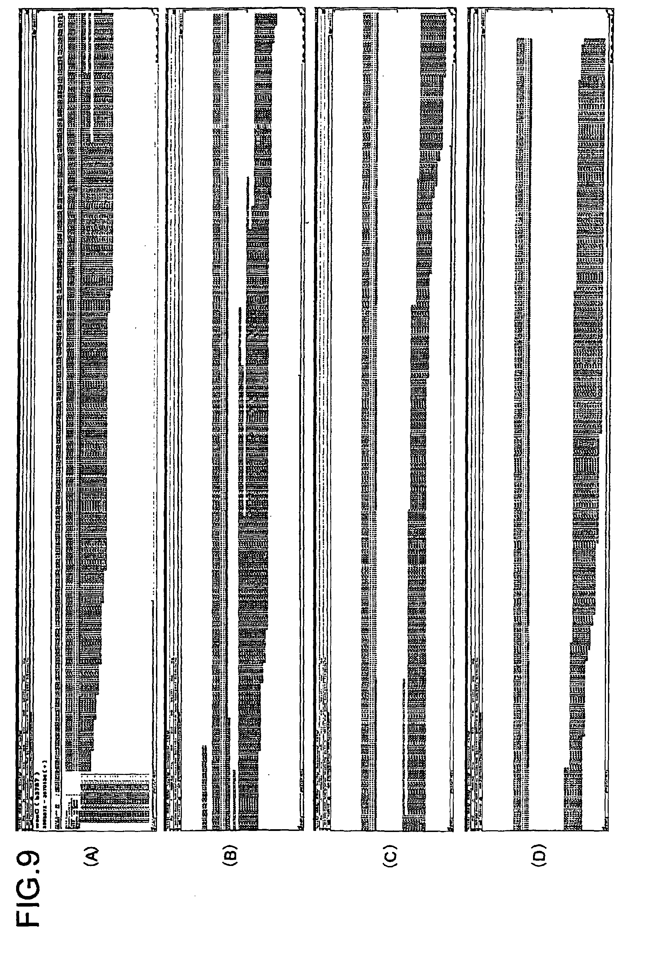

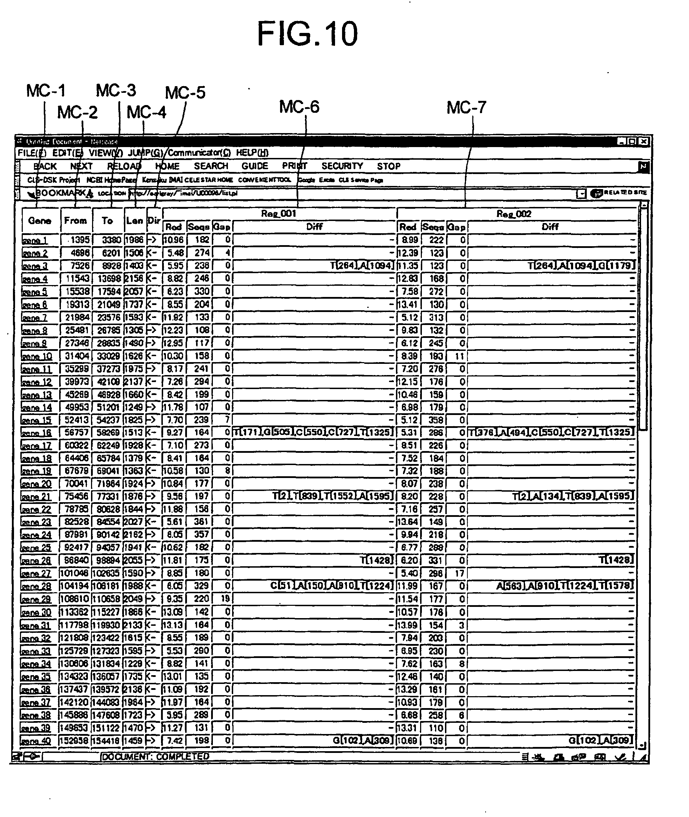

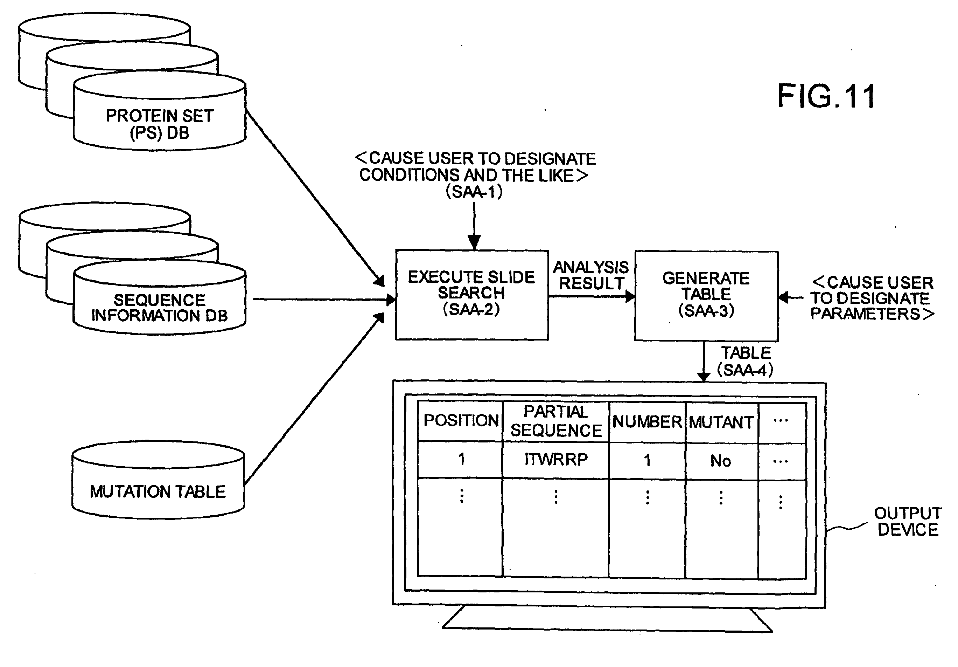

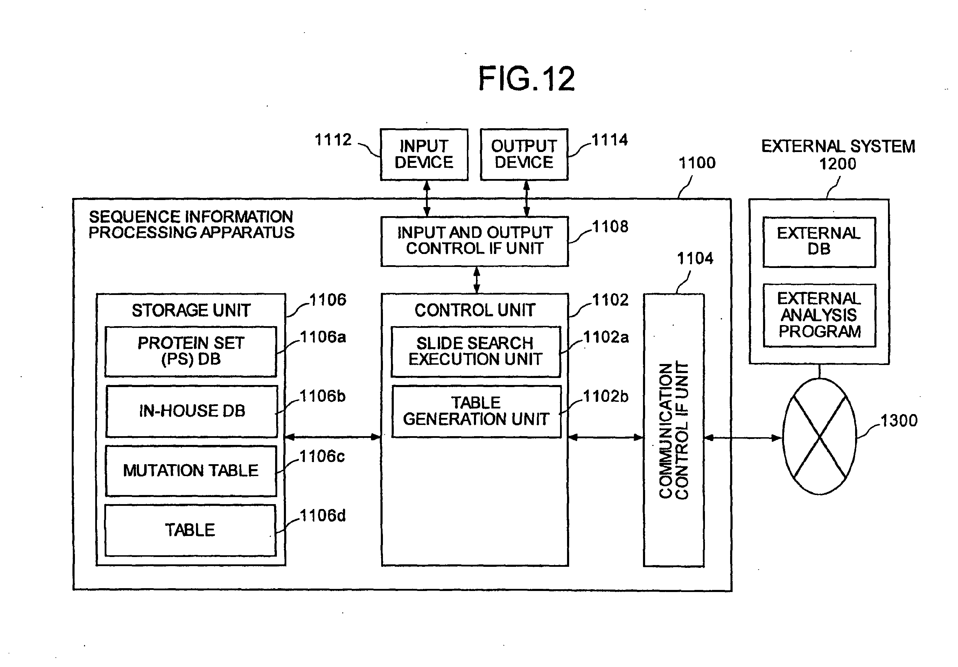

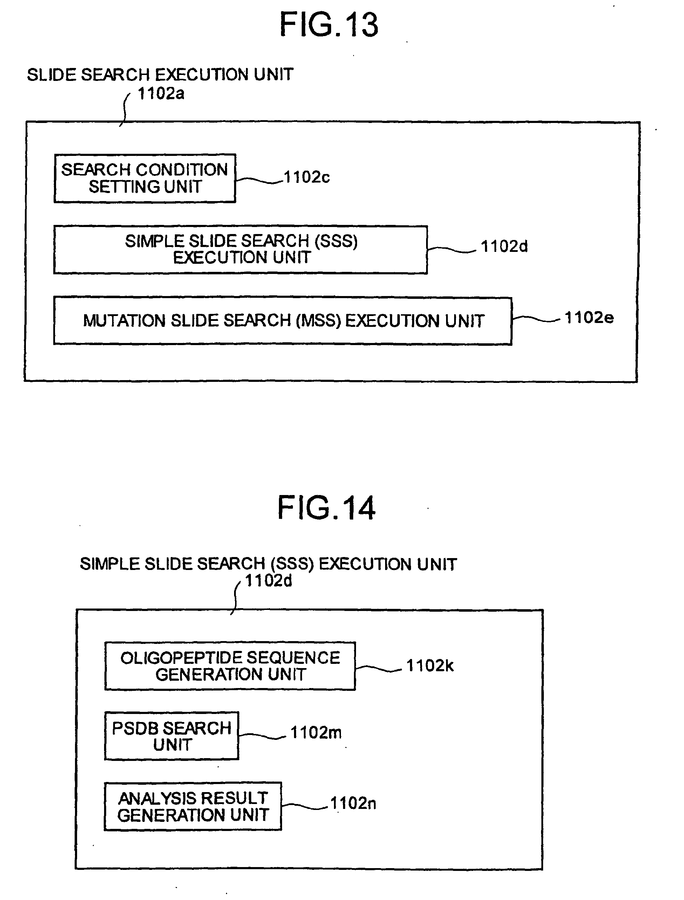

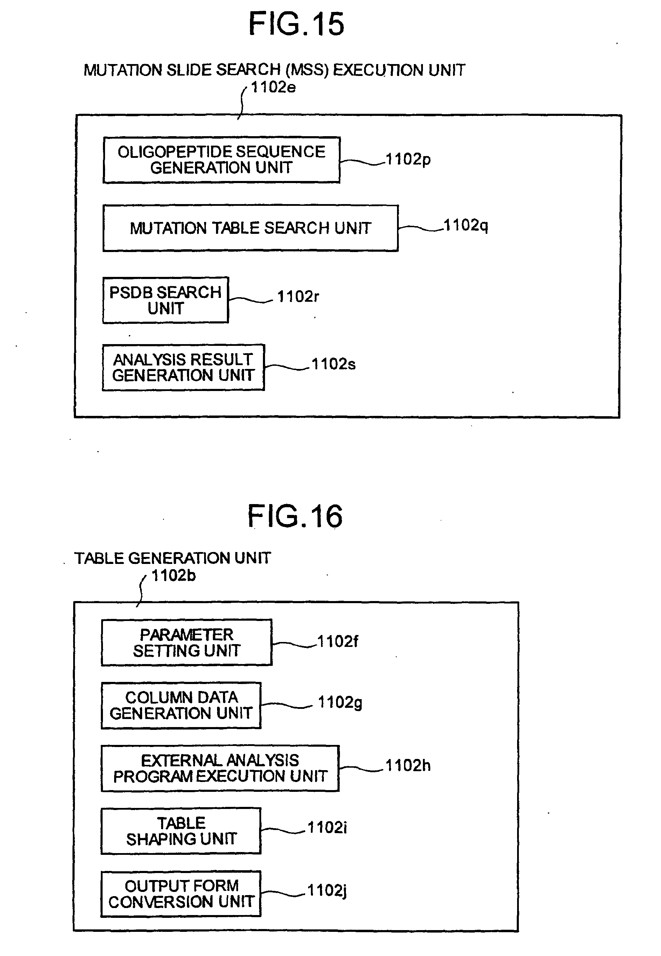

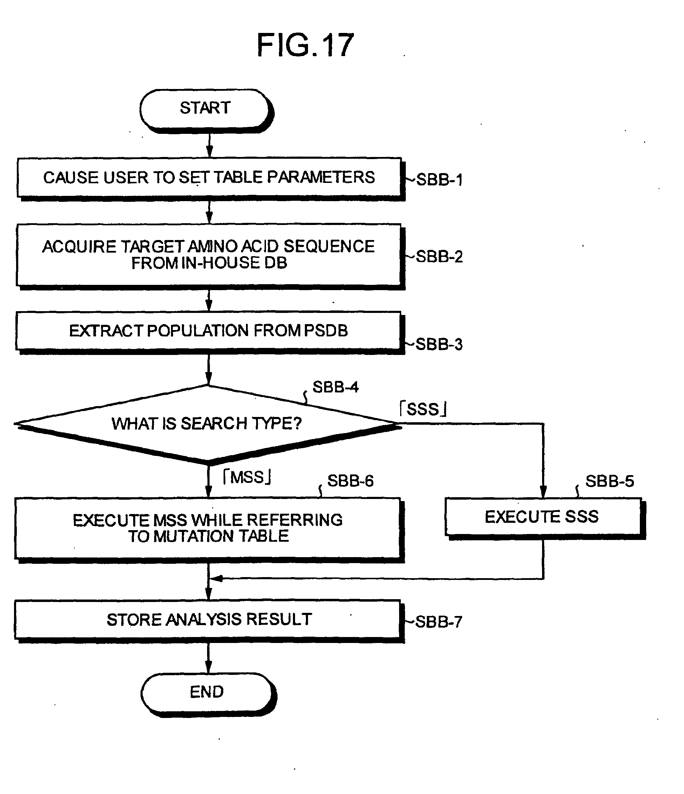

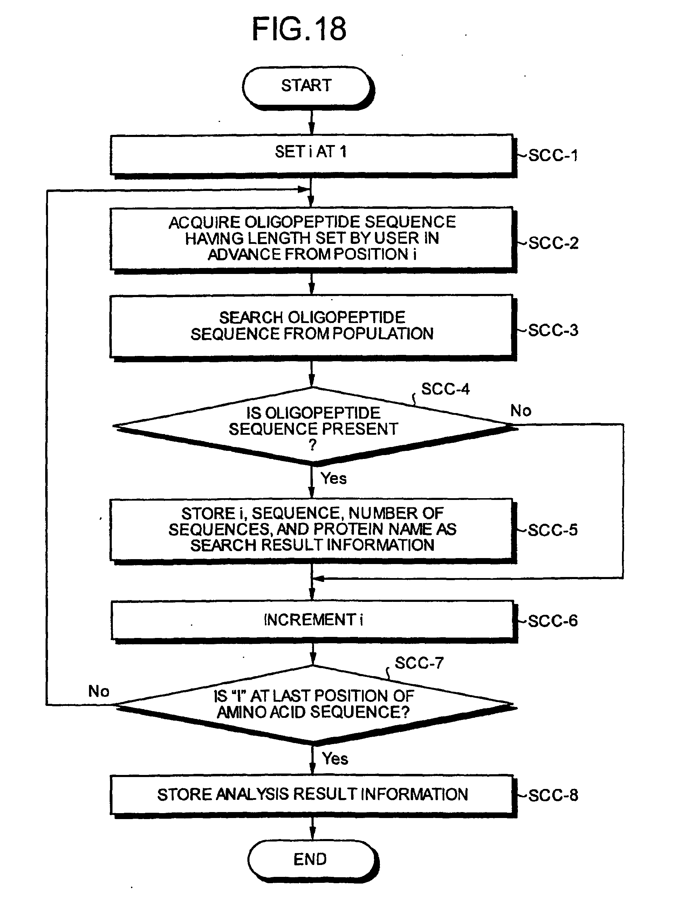

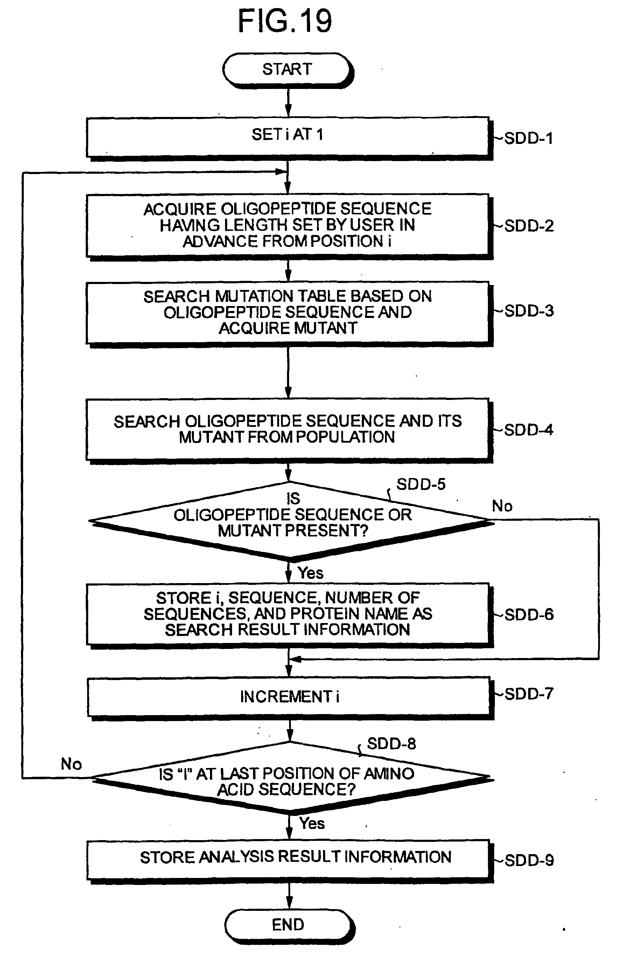

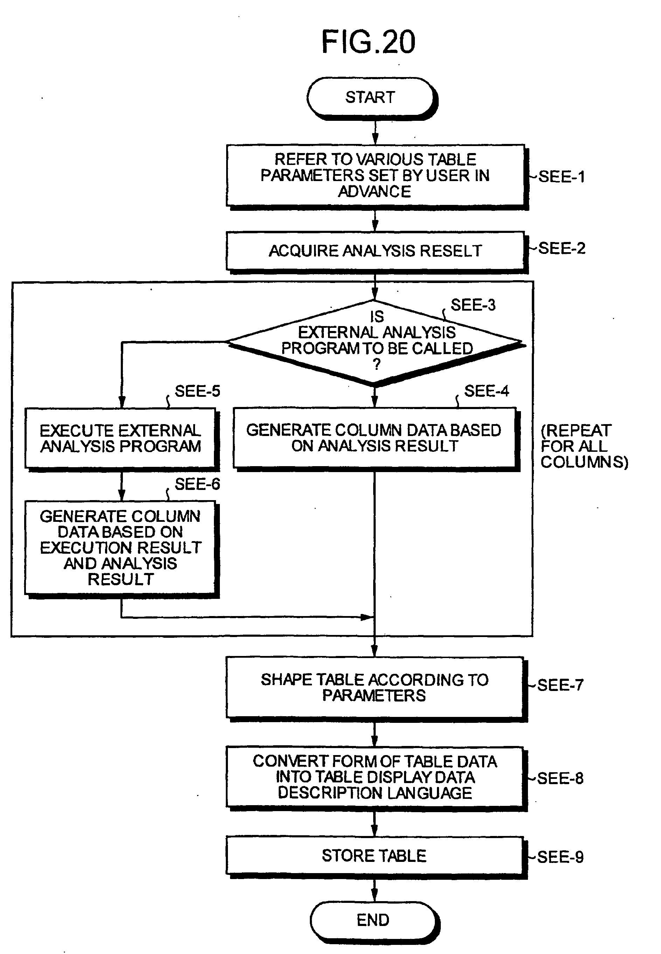

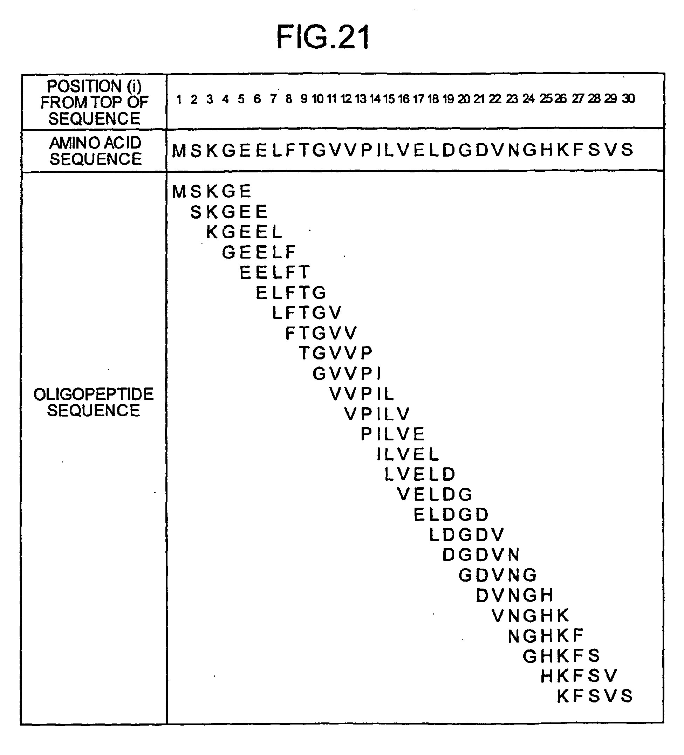

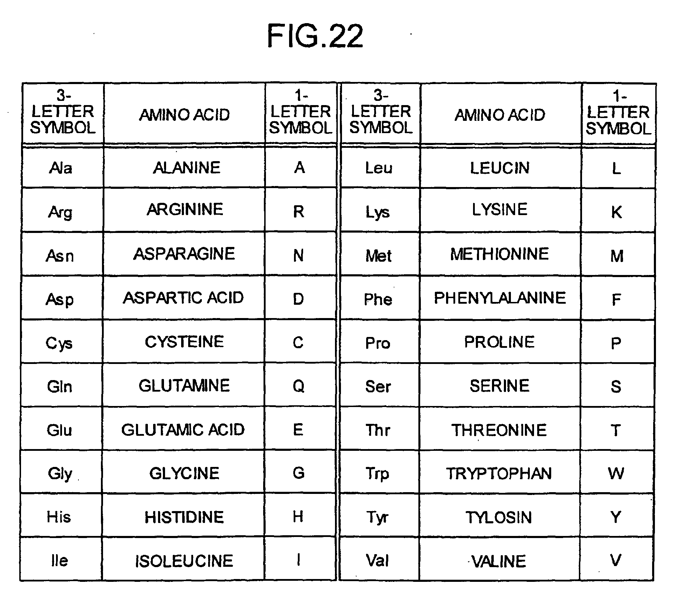

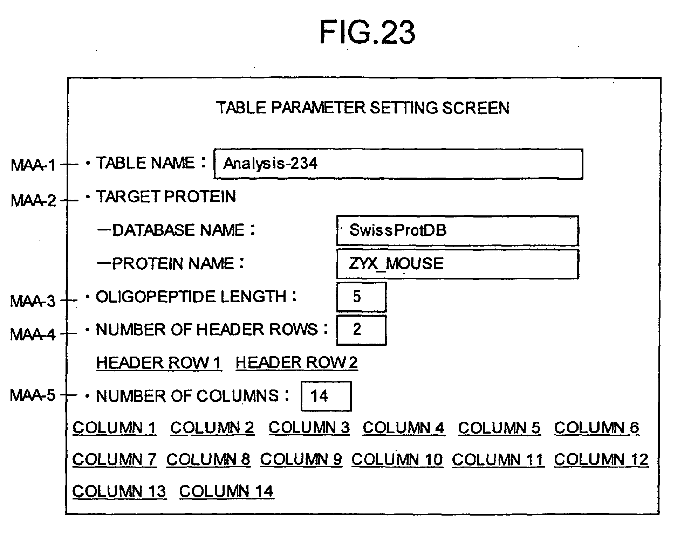

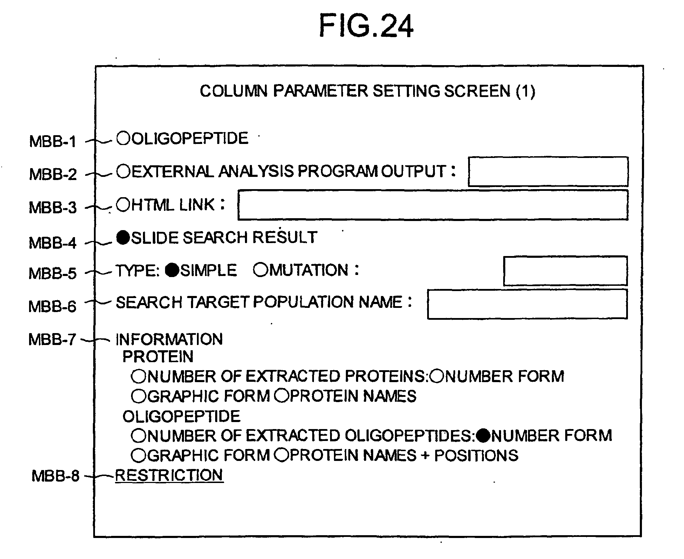

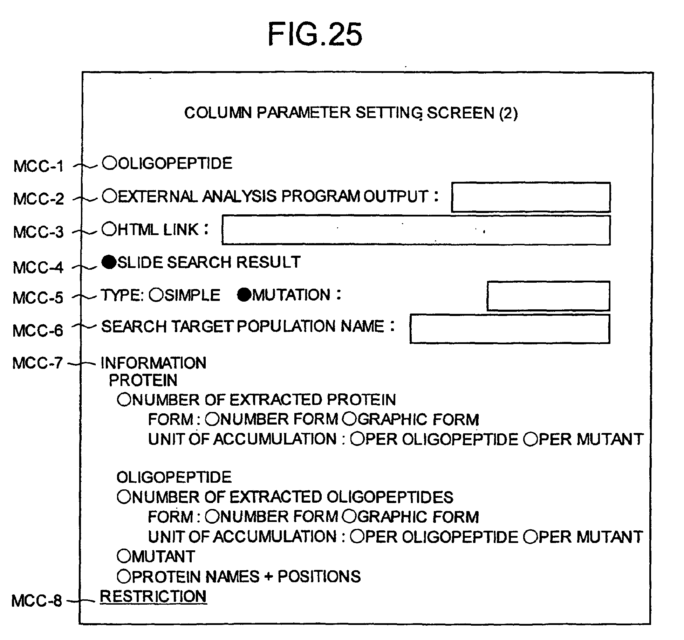

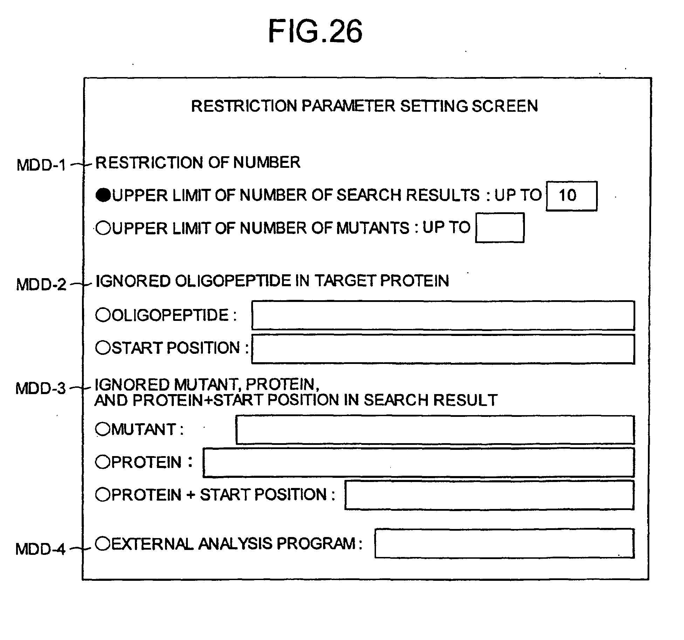

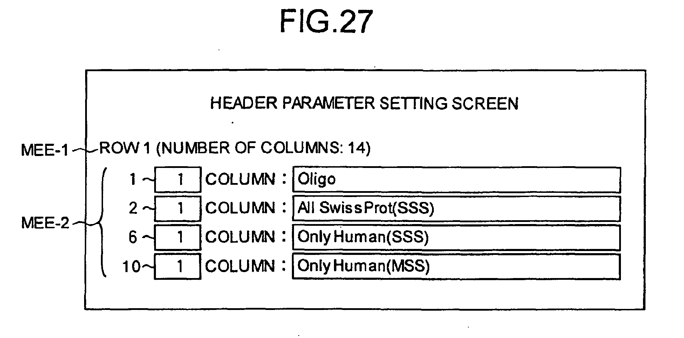

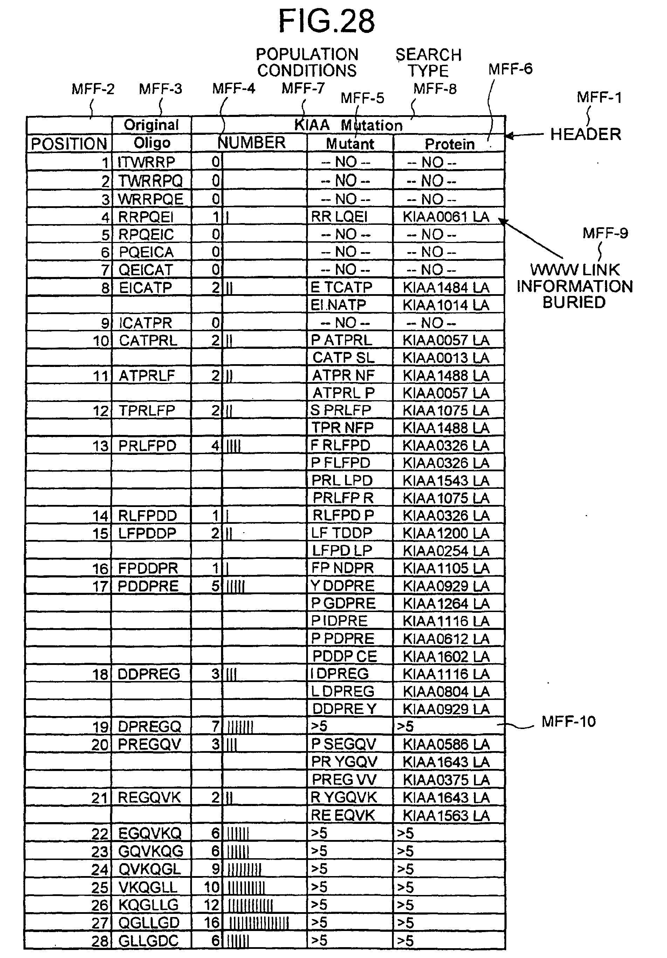

Fig. 1 is a principle block diagram of a basic principle of the present invention; Fig. 2 is a block diagram of one example of a configuration of a system to which the present invention is applied; Fig. 3 is a flowchart of one example of a main processing performed by the system according to one embodiment; Fig. 4 is a flowchart of one example of a region-of-interest selection processing performed by the system according to this embodiment; Fig. 5 is a flowchart of one example of a fragment adoption determination processing performed by the system according to this embodiment; Fig. 6 is a flowchart of one example of a primer sequence determination processing performed by the system according to this embodiment; Fig. 7 depicts one example of a wild strain genome information display screen displayed on a monitor of a mutant sequence analyzing apparatus 100; Fig. 8 depicts one example of a processing result display screen displayed on the monitor of the mutant sequence analyzing apparatus 100; Fig. 9 depicts one example of processing results display screens displayed on monitors in parallel; Fig. 10 depicts one example of a mutation information list display screen about a plurality of mutants displayed on the monitor of the mutant sequence analyzing apparatus 100; Fig. 11 is a principle block diagram of a basic principle of the system; Fig. 12 is a block diagram of one example of a configuration of the system to which the present invention is applied; Fig. 13 is a block diagram of one example of a configuration of a slide search execution unit 1102a; Fig. 14 is a block diagram of one example of a configuration of a simple slide search (SSS) execution unit 1102d; Fig. 15 is a block diagram of one example of a configuration of a mutation slide search (MSS) execution unit 1102e; Fig. 16 is a block diagram of one example of a configuration of a table generation unit 1102b; Fig. 17 is a flowchart of one example of a slide search processing performed by the system according to this embodiment; Fig. 18 is a flowchart of one example of a simple slide search (SSS) processing performed by the system according to this embodiment; Fig. 19 is a flowchart of one example of a mutation slide search (MSS) processing performed by the system according to the embodiment; Fig. 20 is a flowchart of one example of a table generation processing performed by the system according to this embodiment; Fig. 21 is a conceptual view of one example of acquiring a partial sequence (an oligopeptide sequence) having a length set by a user in advance from a position i of a target sequence (an amino acid sequence); Fig. 22 depicts a list of three-letter symbols corresponding to respective one-letter symbols of amino acids used in Fig. 21 and names of the amino acids; Fig. 23 depicts one example of a table parameter setting screen output to an output device 1114 by a processing performed by a search condition setting unit 1102c; Fig. 24 depicts one example of a column parameter setting screen (1) output to the output device 1114 by a processing performed by the search condition setting unit 1102c; Fig. 25 depicts one example of a column parameter setting screen (2) output to the output device 1114 by a processing performed by the search condition setting unit 1102c; Fig. 26 depicts one example of a restriction parameter setting screen output to the output device 1114 by a processing performed by the search condition setting unit 1102c; Fig. 27 depicts one example of the header parameter setting screen output to the output device 1114 by the processing performed by the search condition setting unit 1102c; Fig. 28 depicts one example of a table output to the output device 1114 of a sequence information processing apparatus 1100.

### BEST MODE FOR CARRYING OUT THE INVENTION

(I) Embodiments of a mutant sequence analyzing apparatus, a mutant sequence analyzing method, a program, and a recording medium according to the present invention will be explained in detail with reference to the accompanying drawings. Note that the present invention is not limited to the embodiments.

### [Outline of the Present Invention]

An outline of the present invention will be explained first, and a configuration, processings, and the like of the present invention will then be explained in detail. Fig. 1 is a principle block diagram of a basic principle of the present invention.

The present invention schematically has the following basic features. All DNA's of a mutant of a sequencing target organic species (e.g., a resistant bacterium, a tumor cell, or a virus) are extracted, and shotgun libraries thereof are generated. DNA fragments are sequentially fetched from these libraries, and each fragment is subjected to sequencing using an automated DNA sequencer. Namely, base calling and sequence quality determination are performed by the sequencer.

Fragment sequence information such as base sequences of the respective DNA fragments of the mutant output from the sequencer and the like are stored in a fragment sequence database (at a step SA-1).

Further, original DNA sequence information before mutation (e.g., genome sequence information on a wild strain) stored in an external or internal database is searched, and a region of interest composed by a sequence region of at least a part of the searched original DNA sequence information is selected (at a step SA-2).

As selection of the region of interest, predetermined information (e.g., functions) may be selected from annotation information such as gene function information to thereby automatically select a corresponding sequence region, or a user may select a desired region in the base sequences.

A homology search between the selected region of interest and each fragment sequence information stored in the fragment sequence database is executed by a known scheme such as BLAST or FAST, thereby determining a similarly between the selected region of interest and each fragment sequence information (at a step SA-3).

At this time, estimation may also be executed as to absence of not only sites determined to be similar but also sites determined not to be similar by the homology search. That is, whether each fragment as a query is similar to the wild strain genome not locally but as a whole query may be estimated.

According to a similarly of each fragment obtained as a result of the homology search, it is determined whether to adopt fragments similarities of which exceed a preset similarity as fragment sequence information to be used for mutant sequencing (at a step SA-4).

As the other determination criteria, fragments in which fewer vector sequences are mixed or fragments having appropriate sites hit with respect to the region of interest, lengths, and the like may be selected.

An alignment between each fragment sequence information determined to be adopted and the region of interest is made, whereby each fragment sequence is assigned a position in the region of interest. Based on an alignment result, a consensus sequence of the mutant is determined (at a step SA-5).

In determination of the consensus sequence, a lower sequence quality part may be given a lighter weight using sequence quality information output from the DNA sequencer to thereby improve consensus sequence accuracy.

In addition, the determined consensus sequence is compared with the region of interest to determine mutation information, and a sequence of a mutant part of the mutant is automatically determined (at the step SA-5).

Based on the alignment result, the redundancy of each fragment relative to each base that constitute the region of interest is determined, thereby automatically determining the redundancy that is important information for predicting assembling accuracy and the like (at the step SA-5).

Furthermore, based on the alignment result, a gap part of each fragment relative to the region of interest (a part which cannot be sequenced for the DNA sequence of the mutant, that is, a base sequence part that is present in the original DNA sequence (region of interest) before mutation but that is not present in each fragment (consensus sequence)) is specified.

Information on a known gene locus is made to correspond to the region of interest on the wild strain genome. Using a coding region prediction program or the like, an ORF is identified. A coding region in the ORF is translated to amino acids, and an identification of functions is performed based on an amino acid sequence, as well. Further, annotation is performed based on comparison with genomes of other organic species, thus identifying a so-called orthologue relationship. In parallel to the processing, ORF maps for all genomes are generated. For those gene functions of which have been determined so far, maps classified according to structures and functions are generated, respectively (at the step SA-5).

A processing result including at least one of the region of interest, annotation information on the region of interest (such as amino acid sequence information and function information), the consensus sequence, annotation information on the consensus sequence (such as an ORF region of the consensus sequence, a control region, an amino acid sequence after translation, an original amino acid sequence before mutation, functions after mutation, original functions before mutation, and information on the orthologue relationship), the redundancy, the mutation information (such as a mutant part in each DNA sequence and a mutation content of the mutant part, and a mutant part in each amino acid sequence and a mutation content of the mutant part), the gap information, and the alignment result is displayed (at a step SA-6).

Alternatively, the processing result may be displayed on a plurality of output devices by dividing the processing result by as much as the number of the output devices. By doing so, similarly to an instance of displaying the processing result on a large-sized display (e.g., a horizontally-long display), the processing result can be displayed to correspond to arrangement of a plurality of output devices (e.g., displayed on a virtually horizontally-long output device if a plurality of displays are arranged in a lateral direction). Therefore, an analysis operation can be easily and visually performed when, for example, checking a sequencing status (e.g., the redundancy and the gap), checking a mutant insertion status (e.g., a relative position from any of various loci of the gene or the like, and the number of mutants), checking an influence of the mutation of the base sequence on amino acid translation, and checking genome information on a comparison among a plurality of resistant organisms.

In addition, if both of or one of a sequence region having both of or one of the determined redundancy and a sequence quality, each of which is lower than a preset value, and a sequence region of the determined gap part is present, it is necessary to generate fragments that cover the region and perform sequencing again.

Considering this, according to the present invention, partial sequences located outside of such a region and each having a high specificity in the sequence are automatically extracted and presented to the user. It is thereby possible to specify one site to be annealed by the PCR primer used in the primer walking.

Accordingly, for example, the PCR primer that assists in an experiment for rechecking a mutant-detected site and in an experiment used in the region of interest can be efficiently designed.

Furthermore, PCR primer base sequence candidates are determined according to preset PCR primer selection conditions and presented to the user. By doing so, it is possible to automatically eliminate PCR primers that are annealed to each other to form a dimer, a PCR primer that has partially a higher GC or AT content, a PCR primer that includes a self-complementary sequence and that forms a secondary structure by itself, and a PCR primer having a melting temperature Tm that is a temperature in a state in which 50% of a double-strand DNA forms a hydrogen bond pair and remaining 50% thereof is dissociated, and that is not between an upper limit temperature and a lower limit temperature in a PCR cycle, and the like.

### [System Configuration]

The configuration of a system according to the present invention will first be explained. Fig. 2 is a block diagram of one example of the configuration of the system to which the present invention is applied. Fig. 2 conceptually depicts only constituent elements related to the present invention among all constituent elements of the system. The system is schematically constituted so that a mutant sequence analyzing apparatus 100 and an external system 200 that provides an external database related to sequence information and the like, an external program for a homology search, and the like are communicably connected to each other through a network 300.

In Fig. 2, the network 300 functions to mutually connect the mutant sequence analyzing apparatus 100 and the external system 200, and the network 300 is, for example, the internet.

In Fig. 2, the external system 200 is connected to the mutant sequence analyzing apparatus 100 through the network 300, and functions to provide the user with the external database related to the sequence information and the like and a website for executing the external program for the homology search, the motif search, or the like.

The external system 200 may be constituted as a WEB server, an ASP server, or the like, and a hardware configuration of the external system 200 may be constituted by an information processing apparatus such as a commercially available workstation or personal computer, and peripheries thereof. Respective functions of the external system 200 are realized by a CPU, a disk device, a memory device, an input device, an output device, a communication control device included in the hardware configuration of the external system 200, a program for controlling these devices, and the like.

In Fig. 2, the mutant sequence analyzing apparatus 100 schematically includes a control unit 102, e.g., a CPU, that collectively controls entirety of the mutant sequence analyzing apparatus 100, a communication control interface unit 104 that is connected to a communication device (not shown), e.g., a router, connected to a communication line or the like, an input and output control interface unit 108 that is connected to an input device 112 and an output device 114, and a storage unit 106 that stores various databases, tables, and the like. These units are communicably connected to one another through arbitrary communication paths. Further, this mutant sequence analyzing apparatus 100 is communicably connected to the network 300 through the communication device such as the router and a wired or wireless communication line such as a dedicated line.

The various databases and tables (a fragment sequence database 106a to processing result data 106c) stored in the storage unit 106 are storage units such as fixed disk devices and store various programs, tables, files, databases, webpage files, or the like used for various processings.

Among the constituent elements of the storage unit 106, the fragment sequence database 106a is a fragment sequence storage unit that stores fragment sequence information including information on DNA sequences of the respective fragments of the mutant output from the DNA sequencer, sequence quality information on the DNA sequences, DNA sequences of vectors used in a sequencing experiment, and the like.

A genome information database 106b is a genome information storage unit that stores information on original DNA sequences before mutation (including DNA sequence and amino acid sequence information, information on organic species, information on functions, and the like). The genome information database 106b may be an external database accessed through the Internet, or an in-house database generated by, for example, copying these databases, storing original sequence information and further adding peculiar annotation information and the like.

The processing result data 106c is a processing result data storage unit that stores data on results of various processings performed by the control unit 102.

Further, in Fig. 2, the communication control interface unit 104 controls a communication between the mutant sequence analyzing apparatus 100 and the network 300 (or the communication device such as the router). Namely, the communication control interface unit 104 functions to communicate data with other terminals through communication lines.

In Fig. 2, the input and output control interface unit 108 controls the input device 112 and the output device 114. As the output device 114, a monitor (including a home television set) or a loudspeaker can be used (hereinafter, the output device 114 will be often denoted by the monitor). As the input device 112, a keyboard, a mouse, a microphone, or the like can be used. The monitor together with the mouse also realizes a pointing device function.

In Fig. 2, the control unit 102 includes an internal memory that stores a control program such as an OS (operating system), programs specifying procedures for various processings and the like, and required data. Information processings for executing the various processings are performed based on these programs and the like. The control unit 102 functionally conceptually includes a fragment sequence storage unit 102a, a region-of-interest selection unit 102b, a homology search unit 102c, a fragment adoption determination unit 102d, an alignment processing unit 102e, a redundancy determination unit 102f, a consensus sequence determination unit 102g, a mutation information determination unit 102h, a gap information determination unit 102i, an annotation information determination unit 102j, a processing result display unit 102k, and a primer sequence determination unit 102m.

Among these constituent elements of the control unit 102, the fragment sequence storage unit 102a is a fragment sequence storage unit that stores fragment sequence information on base sequences of DNA fragments of the mutant.

The region-of-interest selection unit 102b is a region-of-interest selection unit that selects a region of interest composed by at least a part of sequence region in the stored original DNA sequence information.

The homology search unit 102c is a homology search unit that determines the similarity between the region of interest selected by the region-of interest selection unit and each fragment sequence information stored in the fragment sequence storage unit.

The fragment adoption determination unit 102d is a fragment adoption determination unit that determines whether to adopt the fragment sequence information as fragment sequence information used to sequence the mutant according to the similarly determined by the homology search unit.

The alignment processing unit 102e is an alignment processing unit that makes an alignment between the fragment sequence information determined to be adopted by the fragment adoption determination unit and the region of interest.

The redundancy determination unit 102f is a redundancy determination unit that determines the redundancy of each fragment relative to each base that constitutes the region of interest based on an alignment result of the alignment processing unit.

The consensus sequence determination unit 102g is a consensus sequence determination unit that determines the consensus sequence of the mutant based on the alignment result of an alignment processing.

The mutation information determination unit 102h is a mutation information determination unit that determines mutation information by comparing the consensus sequence determined by the consensus sequence determination unit with the region of interest.

The gap information determination unit 102i is a gap information determination unit that determines gap information that specifies the gap part of each fragment relative to the region of interest based on the alignment result of the alignment processing unit.

The annotation information determination unit 102j is an annotation information determination unit that determines annotation information that includes at least one of pieces of information on the ORF region of the consensus sequence, the control region, the amino acid sequence after translation, the original amino acid sequence before mutation, the functions after mutation, the original functions before mutation, and the orthologue relationship.

The processing result display unit 102k is a processing result display unit that displays the processing result that includes at least one of the region of interest, the annotation information on the region of interest, the consensus sequence, the annotation information on the consensus sequence, the redundancy, the mutation information, the gap information, and the alignment result.

The primer sequence determination unit 102m is a primer sequence determination unit that determines PCR primer base sequence candidates used to generate fragments including both of or one of a sequence region having both of or one of the redundancy determined by the redundancy determination unit and the sequence quality, each of which is lower than a preset value, and a sequence region of the gap part determined by the gap information determination unit. The primer sequence determination unit 102m includes a partial sequence extraction unit 102n and a primer candidate selection unit 102p.

The partial sequence extraction unit 102n is a partial sequence extraction unit that extracts both of or one of the sequence region having both of or one of the redundancy determined by the redundancy determination unit and the sequence quality, each of which is lower than a preset value, and partial sequences located outside of the sequence region of the gap part determined by the gap information determination unit and each having a high specificity in the sequence.

The primer candidate selection unit 102p is a primer candidate selection unit that determines the PCR primer base sequence candidates according to preset selection conditions among the partial sequences extracted by the partial sequence extraction unit.

Details of the processings performed by the respective units will be explained later.

### [Processings Performed by System]

One example of the processings performed by the system thus constituted according to one embodiment of the present invention will be explained in detail with reference to Figs. 3 to 10.

### [Main Processing]

Details of a main processing will be explained with reference to Fig. 3 and the like. Fig. 3 is a flowchart of one example of the main processing performed by the system according to this embodiment.

By the processing performed by the fragment sequence storage unit 102a, the mutant sequence analyzing apparatus 100 acquires information on fragment sequences such as base sequences of the respective DNA fragments of the mutant output from the sequencer from the input device 112 or via the network 300. In addition, the apparatus 100 stores the fragment sequence information in the fragment sequence database 106a (at a step SB-1).

The mutant sequence analyzing apparatus 100 then execute a region-of-interest selection processing by the processing performed by the region-of-interest selection unit 102b (at a step SB-2). Details of the region-of-interest selection processing will be explained with reference to Figs. 4 and 7. Fig. 4 is a flowchart of one example of the region-of-interest selection processing performed by the system according to this embodiment.

The region-of-interest selection unit 102b accesses the external database of the external system 200, acquires DNA sequence information on, for example, a wild strain and annotation information thereon as the original DNA sequence information before mutation. In addition, the unit 102b stores the DNA sequence information and the annotation information in the genome information database 106b (at a step SC-1). Alternatively, the region-of-interest selection unit 102b may use data on a desired organic species or the like among the data in the external database by copying the data in the internal genome information database 106b in advance.

The region-of-interest selection unit 102b outputs the DNA sequence information and the annotation information on the wild strain to the monitor (at a step SC-2). Fig. 7 depicts one example of a wild strain genome information display screen displayed on the monitor. As shown in Fig. 7, the wild strain genome information display screen includes, for example, a display area MA-1 for displaying a number or a name uniquely identifying a gene region or a control region displayed on a gene map that represents known control regions and gene regions, a display area MA-2 for displaying DNA sequence position information composed by a start position and an end position of each gene or the like, a display area MA-3 for displaying annotation information on the functions and the like corresponding to each gene or the like, an input box MA-4 for inputting a start position of the region of interest, an input box MA-5 for inputting an end position of the region of interest, an input box MA-6 for inputting the annotation information corresponding to the region of interest, and an ENTER button MA-7.

The user inputs desired region-of-interest conditions in the input boxes MA-4 to MA-6 while checking contents of the display areas MA-1 to MA-3, and selects the ENTER button MA-7 using the input device 112 such as the keyboard (at a step SC-3). If so, the region-of-interest selection unit 102b stores the region-of-interest conditions selected by the user in the storage unit 106.

To select the region of interest, the user may designate the start position and the end position on a DNA sequence in the control region, the gene region, or the other arbitrary region, or may designate the annotation information (e.g., the functions of the gene) corresponding to the region of interest.

If the user inputs the annotation information corresponding to the region of interest, the region-of-interest selection unit 102b accesses the genome information database 106b, searches the DNA sequence region corresponding to the annotation information selected by the user from the DNA sequence information on the wild strain, and extracts the DNA sequence region as the region of interest (at a step SC-4). The region-of-interest selection processing is thus finished.

Referring back to Fig. 3, the mutant sequence analyzing apparatus 100 executes the homology search according to the known scheme such as the BLAST between the selected region of interest and each fragment sequence information stored in the fragment sequence database 106a by the processing performed by the homology search unit 102c, thereby determining the similarity (at a step SB-3).

The mutant sequence analyzing apparatus 100 executes a fragment adoption determination processing by the processing performed by the fragment adoption determination unit 102d (at a step SB-4).

Details of the fragment adoption determination processing will be explained with reference to Fig. 5. Fig. 5 is a flowchart of one example of the fragment adoption determination processing performed by the system according to this embodiment.

The fragment adoption determination unit 102d first determines whether to adopt each fragment having the similarity that exceeds a preset similarity as fragment sequence information used to sequence the mutant according to the similarity of the fragment obtained as a result of the homology search (at a step SD-1). Namely, the fragment adoption determination unit 102d eliminates fragments the similarities of which do not satisfy the preset similarity to the region of interest. At this time, the fragment adoption determination unit 102d may also evaluate as to absence of not only sites determined to be similar but also sites determined not to be similar as a result of the homology search. In other words, the fragment adoption determination unit 102d may evaluate whether each fragment as a query is similar to the wild strain genome not locally but as the whole query.

The fragment adoption determination unit 102d conducts the homology search between the vector sequence stored in the fragment sequence database 106a and each fragment sequence, and eliminates a part of the fragment sequence in which the vector sequence is mixed (at a step SD-2).

The fragment adoption determination unit 102d selects fragments having appropriate sites hit with respect to the region of interest, the lengths, and the like (at a step SD-3). Namely, terminal end parts of the fragment are often deteriorated in sequence quality. Therefore, the fragment adoption determination unit 102d determines whether to adopt each fragment based on information on the site and the sequence quality.

Alternatively, the fragment adoption determination unit 102d may calculate a probability that a missequencing occurs due to DNA polymerase for each fragment sequence information so as not to select regions having high probabilities. The fragment adoption determination processing is the finished.

Referring back to Fig. 3, the mutant sequence analyzing apparatus 100 makes an alignment between each fragment sequence information determined to be adopted and the region of interest by the processing performed by the alignment processing unit 102e (at a step SB-5).

The mutant sequence analyzing apparatus 100 determines the redundancy of each fragment relative to each base that constitutes the region of interest based on the alignment result by the processing performed by the redundancy determination unit 102f, thereby automatically determining the redundancy that is important information for predicting assembling accuracy or the like (at a step SB-6).

The mutant sequence analyzing apparatus 100 determines the consensus sequence of the mutant based on the alignment result by the processing performed by the consensus sequence determination unit 102g (at a step SB-7).

The consensus sequence determination unit 102g may give a lighter weight to a lower sequence quality part using the sequence quality information output from the DNA sequencer, thereby improving consensus sequence accuracy. As for a lower redundancy site, in particular, the consensus sequence determination unit 102g gives a higher weight to the sequence information on the higher sequence quality fragment. The quality of the consensus sequence can be thereby enhanced.

The mutant sequence analyzing apparatus 100 determines mutation information by comparing the determined consensus sequence with the region of interest, thereby automatically determining the sequence of the mutant part of the mutant by the processing performed by the mutation information determination unit 102h (at a step SB-8).

The mutant sequence analyzing apparatus 100 specifies the gap part of the fragment relative to the region of interest (the part which cannot be sequenced for the DNA sequence of the mutant, that is, the base sequence part that is present in the original DNA sequence (region of interest) before mutation but that is not present in the fragment (consensus sequence)) by the processing performed by the gap information determination unit 102i (at a step SB-9).

The mutant sequence analyzing apparatus 100 identifies the ORF region using the coding region prediction program or the like by the processing performed by the annotation information determination unit 102j. The annotation information determination unit 102j also translates the ORF region to amino acids and identifies functions from the amino acid sequence. The annotation information determination unit 102j further executes annotation by comparison with genomes of the other organic species, and identifies the so-called orthologue relationship. The annotation information determination unit 102j generates the ORF map for all genomes, and generates maps separated according to structure and function, respectively for those gene functions of which have been determined (at a step SB-10).

The mutant sequence analyzing apparatus 100 displays the processing result including at least one of the region of interest, the annotation information on the region of interest (such as amino acid sequence information and function information), the consensus sequence, the annotation information on the consensus sequence (such as the ORF region of the consensus sequence, the control region, the amino acid sequence after translation, the original amino acid sequence before mutation, the functions after mutation, the original function before mutation, and information on the orthologue relationship), the redundancy, the mutation information (such as the mutant part in each DNA sequence and the mutation content of the mutant part, and the mutant part in each amino acid sequence and a mutation content of the mutant part), the gap information, and the alignment result on the output device 114 by the processing performed by the processing result display unit 102k (at a step SB-11 ).

Fig. 8 depicts one example of a processing result display screen displayed on the monitor of the mutant sequence analyzing apparatus 100. As shown in Fig. 8, the processing result display screen includes, for example, a display area MB-1 for displaying the amino acid sequence information after translation on the gene of the wild strain (the original organic species before mutation), a display area MB-2 for displaying the amino acid sequence information after translation on the gene of the resistant bacteria (mutant), a display area MB-3 for displaying the base sequence information on the DNA of the wild strain, a display area MB-4 for displaying the consensus sequence (if obtained by another experiment) of the resistant strain, a display area MB-5 for displaying the consensus sequence of the resistant strain obtained by an experiment according to this embodiment, a display area MB-6 for displaying mutation information on the base sequence of the DNA of the wild strain and the resistant strain, a display area MB-7 for displaying mutation information on the consensus sequence of the resistant strain obtained by another experiment and the resistant strain obtained by the experiment according to this embodiment, a display area MB-8 for displaying the redundancy of the fragment, and a display area MB-9 for displaying the base sequence information on the DNA of each fragment.

The processing result display unit 102k may display the processing result on a plurality of output devices 114 by dividing the processing result by as much as the number of output devices as shown in Figs. 9. Fig. 9 depicts one example of processing results display screens displayed on the monitors in parallel. In Fig. 9, (A) to (D) show display contents of the respective output devices 114 if the processing result is displayed on a plurality of (four in the example of Fig. 9) output devices 114 by dividing the processing result to a plurality of (four in the example of Fig. 9) segments.

By doing so, similarly to an instance of displaying the processing result on a large-sized display (e.g., a horizontally-long display), the processing result can be displayed to correspond to arrangement of a plurality of output devices (e.g., displayed on a virtually horizontally-long output device if a plurality of displays are arranged in a lateral direction). Therefore, an analysis operation can be easily and visually performed when, for example, checking the sequencing status (e.g., the redundancy and the gap), checking the mutant insertion status (e.g., the relative position from any of various loci of the gene or the like, and the number of mutants), checking the influence of the mutation of the base sequence on amino acid translation and the like, and checking genome information on the comparison among a plurality of resistant organisms.

Fig. 10 depicts one example of a mutation information list display screen about a plurality of mutants displayed on the monitor of the mutant sequence analyzing apparatus 100. As shown in Fig. 10, the mutation information list display screen includes, for example, a display area MC-1 for displaying a name or a number for uniquely identifying each gene of the wild strain, a display area MC-2 for displaying a start position of the gene on the DNA sequence, a display area MC-3 for displaying an end position of the gene on the DNA sequence, a display area MC-4 for displaying a length of the gene, a display area MC-5 for displaying a sequencing direction, a display area MC-6 for displaying information on a resistant strain 1, and a display area MC-7 for displaying information on a resistant strain 2.

In Fig. 10, an instance in which the number of resistant strains is 2 is explained. In addition, the information on each resistant strain is composed by the redundancy of the fragment (Red), the number of fragment sequences (Seqs), the number of gaps during determination of the consensus sequence (Gap), and the mutation information (Diff). The mutation information is composed by substituent bases and positions of the bases (put in parentheses after the respective bases). The main processing is thus finished.

### [Primer Sequence Determination Processing]

Details of a primer sequence determination processing will be explained with reference to Fig. 6. Fig. 6 is a flowchart of one example of the primer sequence determination processing.

If both of or one of the sequence region having both of or one of the redundancy determined by the processing above and the sequence quality, each of which is lower than a preset value, and the sequence region of the determined gap part is present (at a step SE-1), it is necessary to generate fragments that cover the region and perform sequencing again. Therefore, the mutant sequence analyzing apparatus 100 automatically extracts partial sequences located outside of such a region and each having a high specificity in the sequence by the processing performed by the partial sequence extraction unit 102n (at a step SE-2).

As extraction of the high specificity sequence part performed by the partial sequence extraction unit 102n, a local alignment of each part of the DNA sequence of the mutant relative to the whole sequence (consensus sequence) is made, and the part that satisfies a certain homology score is excluded as a part for which a similar sequence is present in the other part, thereby extracting partial sequences for each of which the similar sequence is not present in the other part (which has a high specificity).

The mutant sequence analyzing apparatus 100 selects sequence partial regions having G or C terminals from among the extracted sequence part regions (at a step SE-3) by the processing of the primer candidate selection unit 102p. It is thereby possible to intensify a hybridization strength of a PCR primer.

The primer candidate selection unit 102p selects the sequence partial regions each of which does not include complementary sequences from among the extracted sequence partial regions (at a step SE-4). It is thereby possible to eliminate primers that constitute secondary structures by themselves.

The primer candidate selection unit 102p selects sequence partial regions each of which does not include a sequence having four or more consecutive G or C from among the extracted sequence partial regions (at a step SE-5). It is thereby possible to prevent tetraplex.

The primer candidate selection unit 102p selects sequence partial regions each of which has a melting point Tm between the upper limit temperature and the lower limit temperature used in a PCR cycle from among the extracted sequence partial regions (at a step SE-6). It is thereby possible to prevent use of primers that inhibit PCR reaction.

Further, the primer candidate selection unit 102p automatically eliminates a sequence partial region in which primers are annealed to each other to form a dimer, a sequence partial region in which a primer partially has a higher GC or AT content, a sequence partial region in which a primer includes a self-complementary sequence and forms a secondary structure by itself, a sequence partial region in which a primer having the melting temperature Tm that is the temperature in a state in which 50% of a double-strand DNA forms a hydrogen bond pair and remaining 50% thereof is dissociated, and that is not between the upper limit temperature and the lower limit temperature in the PCR cycle, and the like. The primer candidate selection unit 102p thereby determines PCR primer base sequence candidates.

The primer candidate selection unit 102p determines the PCR primer base sequence candidates among the extracted partial sequences according to the preset PCR primer selection conditions, and displays the candidates on the output device 114, thereby presenting the user with information assisting in generation of PCR primers (at a step SE-7). The primer sequence determination processing is thus finished.

### [Other Embodiments]

While exemplary embodiments of the present invention have been explained above, the invention may be also practiced by various embodiments other than the above-described ones, within the technical scope of the appended claims.

For example, when a mutant site is specified and extracted, a region including a site in which a mutant can be detected more clearly in the whole genome as the mutant region between the wild strain and the variant.

Further, a statistical analysis of mutant sites and the like for a plurality of variants relative to the wild strain (a relative analysis between a phenotype and a mutant) may be output.

Furthermore, while the instance in which the mutant sequence analyzing apparatus 100 performs the processing in a standalone fashion has been explained, the system may also be constituted such that the mutant sequence analyzing apparatus 100 performs each processing in response to a request from a client terminal provided separately from the mutant sequence analyzing apparatus 100, and transmits the processing result to the client terminal.

Of the respective processing explained in the embodiments, all or a part of the processing explained as being performed automatically may be performed manually, or all or a part of the processing explained as being performed manually may be performed automatically in a known method.

In addition, information that includes the processing procedure, the control procedure, specific names, various kinds of registered data, and parameters such as search conditions, shown in the specification or in the drawings, as well as screen examples and database configurations can be optionally changed, unless otherwise specified.

As for the mutant sequence analyzing apparatus 100, the respective constituent elements of the apparatus shown in the drawings are functionally conceptual and are not necessarily constituted physically as shown in the drawings.

For example, all or an arbitrary part of the processing functions of the respective units or devices included in the mutant sequence analyzing apparatus 100 can be realized by the CPU (Central Processing Unit) and a program interpreted and executed by the CPU, or can be realized as wired logic-based hardware. The program is recorded in a recording medium to be explained later, and mechanically read by the mutant sequence analyzing apparatus 100 if necessary.

Namely, a computer program for transmitting a command to the CPU in cooperation with the OS (Operating System) to allow performing various processings is recorded in the storage unit 106 such as a ROM or a HD. This computer program is executed by being loaded into a RAM or the like, and the computer program as well as the CPU constitutes the control unit 102. This computer program may be recorded in an application program server connected to the mutant sequence analyzing apparatus 100 through the arbitrary network 300. All or a part of the computer program can be downloaded if necessary.

The program according to the present invention can be stored in a computer readable recording medium. It is assumed herein that the "recording medium" includes arbitrary "portable physical medium" such as a flexible disk, a magneto-optical disk, a ROM, an EPROM, an EEPROM, a CD-ROM, an MO, and a DVD, arbitrary "fixed physical medium" such as a ROM, a RAM, and an HD included in various computer systems, and "communication medium" that temporarily holds the program such as a communication line or a carrier wave, which is used when the program is transmitted through the network represented by a LAN, a WAN, or the internet.

Furthermore, the "program" signifies a data processing method described in an arbitrary language or by an arbitrary description method, and a form of the program is not limited to a source code, a binary code, or the like. It is noted that the "program" is not always limited to the program constituted singularly. Examples of the "program" include a program that is distribution-structured as a plurality of modules or libraries, or attains its functions in cooperation with another program represented by the OS (Operating System). As for specific configurations and reading procedures for reading the recording medium by the respective devices explained in the embodiment as well as installation procedures and the like after reading, well-known configurations and procedures can be also used.

The various databases and the like (the fragment sequence database 106a to the processing result data 106c) stored in the storage unit 106 are storage units such as memory devices, e.g., the RAM and the ROM, fixed disk devices such as hard disks, flexible disks, and optical disks. These databases and the like store various programs, tables, files, databases, webpage files, and the like used for various processings, respectively.

The mutant sequence analyzing apparatus 100 may be realized by connecting peripherals such as a printer, a monitor, and an image scanner to an information processing apparatus such as a known information processing terminal, e.g., a personal computer or a workstation, and installing software (including the program, the data, and the like) for realizing the method according to the present invention into the information processing apparatus.

Furthermore, specific forms of distribution or integration of the mutant sequence analyzing apparatus 100 are not limited to those shown in the drawings. The apparatus 100 can be constituted by distributing or integrating all or a part of the apparatus 100 either functionally or physically in arbitrary units according to various loads and the like. For example, the respective databases may be constituted independently as independent database devices, or part of the processings may be realized using a CGI (common gateway interface).

Additionally, the network 300 functions to connect the mutant sequence analyzing apparatus 100 to the external system 200. The network 300 may include, for example, any one of the internet, an Intranet, a LAN (which may be wired or wireless), a VAN, a personal computer communication network, a public telephone network (which may be analog or digital), a dedicated line network (which may be analog or digital) a CATV network, a portable line exchange network and portable packet exchange network of an IMT2000, a GSM, or PDC/PDC-P, a wireless call network, a local wireless network such as Bluetooth, a PHS network, and satellite communication networks such as CS, BS, and ISDB. Namely, the system according to the present invention can transmit and receive various pieces of data through the arbitrary network regardless of the network to be wired or wireless.

As explained so far in detail, according to the present invention, fragment sequence information on a base sequence of each fragment of a mutant is stored, original DNA sequence information before mutation is stored, a region of interest composed by a sequence region of at least a part of the stored original DNA sequence information stored is selected, a similarity between the selected region of interest and the stored fragment sequence information is determined, it is determined whether to adopt the fragment sequence information as the fragment sequence information used for sequencing the mutation according to the determined similarity, an alignment between the fragment sequence information determined to be adopted and the region of interest is made, and a consensus sequence of the mutant is determined based on an alignment result. Therefore, it is possible to provide the mutant sequence analyzing apparatus, the mutant sequence analyzing method, the program, and the recording medium capable of efficiently determining the DNA sequence of the mutant part by specifying the region of interest predicted to undergo mutation, and generating the consensus sequence from the fragments of the mutant assigned to the region of interest.

Further, according to the conventional art, it is necessary to perform the operation for determining the sequences of all genomes of the mutant. Accordingly, it is necessary to perform adjustment of libraries, generation of fragments, and the like for all the genomes, thereby casting heavy burden of the wet experiment on the conventional analysis. In addition, after executing an alignment of a vast number of fragment sequences on the calculator for assembling, it is necessary to edit and check the base sequences. Accordingly, a calculator-side operational burden is also cast on the conventional analysis. According to the apparatus of the present invention, it is unnecessary to determine the sequences of all genomes, and it suffices only to experiment and calculate only the region of interest. Therefore, it is possible to provide the mutant sequence analyzing apparatus, the mutant sequence analyzing method, the program, and the recording medium capable of efficiently utilizing various resources, and promptly and accurately determining the DNA sequence of the mutant.

Furthermore, the conventional assembling requires overlap (redundancy) among the fragment sequences. Accordingly, accurate assembling cannot be expected on small-overlap parts, i.e., low redundancy parts or low fragment sequence quality parts of the fragment sequence. According to the apparatus of the present invention, the consensus sequence is generated using the fragments assigned to the original DNA sequence information before mutation even for a part in which it is difficult to perform assembling. Therefore, it is possible to provide the mutant sequence analyzing apparatus, the mutant sequence analyzing method, the program, and the recording medium capable of identifying or estimating the base sequence with high accuracy.

According to the present invention, mutation information is determined by comparing the determined consensus sequence with the region of interest. Therefore, it is possible to provide the mutant sequence analyzing apparatus, the mutant sequence analyzing method, the program, and the recording medium capable of automatically determining the sequence of the mutant part of the mutant.

According to the present invention, a redundancy of each fragment relative to each base that constitutes the region of interest is determined based on an alignment result. Therefore, it is possible to provide the mutant sequence analyzing apparatus, the mutant sequence analyzing method, the program, and the recording medium capable of automatically determining the redundancy that is important information for predicting assembling accuracy and the like.

According to the present invention, gap information that specifies a gap part of each fragment relative to the region of interest is determined based on an alignment result. Therefore, it is possible to provide the mutant sequence analyzing apparatus, the mutant sequence analyzing method, the program, and the recording medium capable of automatically determining the part which cannot be sequenced for the DNA sequence of the mutant, that is, a base sequence part that is present in the original DNA sequence (region of interest) before mutation but that is not present in each fragment (consensus sequence).

According to the present invention, a processing result including at least one of the region of interest, annotation information on the region of interest, the consensus sequence, annotation information on the consensus sequence, the redundancy, the mutation information, the gap information, and the alignment result is displayed. Therefore, it is possible to provide the mutant sequence analyzing apparatus, the mutant sequence analyzing method, the program, and the recording medium capable of accelerating discovering a new biological knowledge by the user who checks such useful information.

According to the present invention, the annotation information on the consensus sequence includes information on at least one of an ORF region of the consensus sequence, a control region, an amino acid sequence after translation (e.g., substitutional mutation, and frame shift mutation caused by insertion or deletion), a function after the mutation (e.g., a function change caused by mutation of the gene region or that of the control region, and function correlation analysis for a plurality of gene regions having mutation correlation), and an orthologue relationship (e.g., analysis of genes of different types that hold the same mutation, and strain analysis). Therefore, it is possible to provide the mutant sequence analyzing apparatus, the mutant sequence analyzing method, the program, and the recording medium capable of accelerating discovering a new biological knowledge by the user who checks these pieces of useful information by searching a known genome information database such as cDNA database, searching a similar sequence or function among cells and the like of the same type or different types, and determining information on these cells and the like.

According to the present invention, the processing result is displayed on a plurality of output devices by dividing the processing result by as much as the number of the output devices. Therefore, it is possible to provide the mutant sequence analyzing apparatus, the mutant sequence analyzing method, the program, and the recording medium capable of displaying the processing result to correspond to arrangement of a plurality of output devices (e.g., displayed on a virtually horizontally-long output device if a plurality of displays are arranged in a lateral direction) similarly to an instance of displaying the processing result on a large-sized display (e.g., a horizontally-long display).

Namely, according to the present invention, it is possible to provide the mutant sequence analyzing apparatus, the mutant sequence analyzing method, the program, and the recording medium capable of easily and visually performing an analysis operation when, for example, checking a sequencing status (e.g., a redundancy and a gap), checking a mutant insertion status (e.g., a relative position from any of various loci of the gene or the like, and the number of mutants), checking an influence of the mutation of the base sequence on amino acid translation and the like, and checking genome information on a comparison among a plurality of resistant organisms.

According to the present invention, base sequence candidates of a PCR primer used to generate the each fragment that includes both of or one of a sequence region having both of or one of the determined redundancy and the sequence quality, each of which is lower than a preset value, and a sequence region of the determined gap part are determined. Therefore, it is possible to provide the mutant sequence analyzing apparatus, the mutant sequence analyzing method, the program, and the recording medium capable of, as a result of sequencing, automatically designing the PCR primer used to generate fragments that cover the gap part and the small fragment overlap part, and realizing automation of operation and improvement of efficiency.

According to the present invention, partial sequences located outside of both of or one of the sequence region having both of or one of the determined redundancy and the sequence quality, each of which is lower than the preset value, and the sequence region of the determined gap part, and each having a high specificity in each of the sequences are extracted. Therefore, it is possible to provide the mutant sequence analyzing apparatus, the mutant sequence analyzing method, the program, and the recording medium capable of specifying one site to be annealed by the PCR primer used in the primer walking.

Furthermore, it is thereby possible to provide the mutant sequence analyzing apparatus, the mutant sequence analyzing method, the program, and the recording medium capable of efficiently designing the PCR primer for supporting an experiment of rechecking of the mutant detected site, an experiment using the region of interest, and the like instead of specifying the low redundancy site or the gap part.

According to the present invention, the base sequence candidates of the PCR primer are determined among the extracted partial sequences according to preset selection conditions. Therefore, it is possible to provide the mutant sequence analyzing apparatus, the mutant sequence analyzing method, the program, and the recording medium capable of determining the base sequence candidates of the PCR primer after eliminating, for example, PCR primers that are annealed to each other to form a dimer, a PCR primer that has partially a higher GC or AT content, a PCR primer that includes a self-complementary sequence and that forms a secondary structure by itself, a PCR primer having a melting temperature Tm that is a temperature in a state in which 50% of a double-strand DNA forms a hydrogen bond pair and remaining 50% thereof is dissociated, and that is not between an upper limit temperature and a lower limit temperature in a PCR cycle, and the like.

Moreover, according to the present invention, each of the partial sequences is a base sequence having the number of bases equal to or greater than 15 and equal to or smaller than 30 (preferably 20 to 25). Therefore, the PCR can be efficiently executed. Namely, if the number of bases is equal to or smaller than 14, it is highly likely that the partial sequence cannot be satisfactorily annealed to a template. If the number of bases is equal to or greater than 31, a probability of misannealing increases. Therefore, it is possible to provide the mutant sequence analyzing apparatus, the mutant sequence analyzing method, the program, and the recording medium capable of determining a high quality primer sequence by specifying an appropriate number of bases.

(II) Exemplary embodiments of a sequence information processing apparatus, a sequence information processing method, a program, and a recording medium according to the present invention will be explained in detail with reference to the accompanying drawings. Note that the present invention is not limited to the embodiments.

While in the embodiments below, the present invention will be explained by an example applying a system that displays sequence information on oligopeptides, the invention may also apply any systems that display partial sequences of sequence information on all kinds.

### [Outline of System]

An outline of a system according to the present invention will be explained first, and a configuration, processings, and the like of the system will then be explained in detail. Fig. 11 is a principle block diagram depicts an example of a basic principle of the system. Fig. 11 conceptually depicts only constituent elements related to the present invention among all constituent elements of the system.

The system is a system that allows a user to output an analysis result of oligopeptide bases related to an amino acid sequence of a target protein in a desired table form. The user first designates the amino acid sequence of the target protein from a sequence information database. To designate the amino acid sequence, pieces of information that can be designated may be output onto a screen so that the user selects a desired amino acid sequence from among the output information, or the user may designate the desired amino acid sequence by directly specifying the desired amino acid sequence and executing a corresponding script or the like. Similarly, in the following explanation, any known designation method may be used as the user designation method.

The sequence information database may be an external amino acid sequence database, e.g., SWISS-PROT, TrEMBL, PIR, GenPept, or PRF/SEQDB, accessed through the Internet, or may be an in-house database generated by, for example, copying these databases, storing original sequence information, and further adding peculiar annotation information and the like.

The user designates conditions for extracting a population, which is a search target, from a protein set (PS) generated in advance. The PSDB is a database generated by collecting sequence information on proteins that possess specific properties, e.g., a PSDB in which sequence information on human proteins are stored. The PSDB may be generated by, for example, extracting sequence information coincident with a predetermined keyword (e.g., "human") from the external database or the in-house database by a natural language search or the like.

Furthermore, the user designates whether a search range covers up to mutants. The mutant is an individual in which a predetermined oligopeptide sequence may possibly undergo mutation. Each oligopeptide sequence and a mutant sequence thereof are stored in a mutation table in advance while making them correspond to each other.

The user thus completes designating the conditions and the like for a slide search (at a step SAA-1). If so, the slide search is executed in the sequence information processing apparatus according to the present invention (at a step SAA-2). The slide search means performing various analyses for the target sequence by dividing the target sequence to partial sequences each having a length designated by the user in advance from a top of the sequence, and searching the partial sequences from sequence information on the population. A basic scheme for the slide search will be explained later.

An analysis result of the slide search as well as related information and the like is formed into a table according to an output form set by parameters or the like designated by the user in advance (at a step SAA-3).

The generated table is output to an output device such as a display device (at a step SAA-4). Consequently, information on partial sequence information necessary for the user can be displayed or the like in the table form so that the user can easily see.

### [System Configuration]

The configuration of the system for embodying these basic features will be explained.

### [System Configuration]

The configuration of the system will first be explained. Fig. 12 is a block diagram of one example of the configuration of the system to which the present invention is applied. Fig. 12 conceptually depicts only constituent elements related to the present invention among all constituent elements of the system. The system is schematically constituted so that a sequence information processing apparatus 1100 that is a sequence information processing apparatus processing the sequence information and an external system 1200 that provides a database related to the sequence information and the like, a program for a homology search, and the like are communicably connected to each other through a network 1300.

In Fig. 12, the network 1300 functions to mutually connect the sequence information processing apparatus 1100 and the external system 1200, and the network 1300 is, for example, the Internet.

In Fig. 12, the external system 1200 is connected to the sequence information processing apparatus 1100 through the network 1300, and functions to provide the user with the external database related to the sequence information and the like and a website for executing the external program for the homology search, the motif search, or the like.

The external system 1200 may be constituted as a WEB server, an ASP server, or the like, and a hardware configuration of the external system 1200 may be constituted by an information processing apparatus such as a commercially available workstation or personal computer, and peripheries thereof. Respective functions of the external system 1200 are realized by a CPU, a disk device, a memory device, an input device, an output device, a communication control device included in the hardware configuration of the external system 1200, a program for controlling these devices, and the like.

In Fig. 12, the sequence information processing apparatus 1100 schematically includes a control unit 1102, e.g., a CPU, that collectively controls entirety of the sequence information processing apparatus 1100, a communication control interface unit 1104 that is connected to a communication device (not shown), e.g., a router, connected to a communication line or the like, an input and output control interface unit 1108 that is connected to an input device 1112 and an output device 1114, and a storage unit 1106 that stores various databases and tables (a protein set (PS) database 1106a to a table 1106d). These units are communicably connected to one another through arbitrary communication paths. Further, this sequence information processing apparatus 1100 is communicably connected to the network 1300 through the communication device such as the router and a wired or wireless communication line such as a dedicated line.

The various databases and tables (the protein set (PS) database 1106a to the table 1106d) stored in the storage unit 1106 are storage units such as fixed disk devices and store various programs, tables, files, databases, webpage files, or the like used for various processings.

Among the constituent elements of the storage unit 1106, the protein set (PD) database 1106a is a database generated by collecting sequence information on proteins that possess specific properties. An in-house database 1106b is a sequence information storage unit that stores base or amino acid sequence information and sequence related information related to the sequence information while making them corresponding to each other. The sequence related information includes information on at least one of a name of a sequence, a name of an organism from which the sequence is derived, a name of a corresponding protein, functions of the protein, and an address of a related database.

A mutation table 1106c is a mutant sequence information storage unit that stores the sequence information and mutant sequence information on a mutant sequence to which the sequence information may possibly be mutated while making them correspond to each other. The table 1106d is information generated by using partial sequence information on partial sequences and the analysis result obtained by the slide search according to the user's designated output form.

Further, in Fig. 12, the communication control interface unit 1104 controls a communication between the sequence information processing apparatus 1100 and the network 1300 (or the communication device such as the router). Namely, the communication control interface unit 1104 functions to communicate data with other terminals through communication lines.

In Fig. 12, the input and output control interface unit 1108 controls the input device 1112 and the output device 1114. As the output device 1114, a monitor (including a home television set) or speakers can be used (hereinafter, the output device will be often denoted by the monitor). As the input device 1112, a keyboard, a mouse, a microphone, or the like can be used. The monitor together with the mouse also realizes a pointing device function.

In Fig. 12, the control unit 1102 includes an internal memory that stores a control program such as an OS (Operating System), programs specifying procedures for various processings and the like, and required data. Information processings for executing the various processings are performed based on these programs and the like. The control unit 1102 functionally conceptually includes a slide search execution unit 1102a and a table generation unit 1102b.

Among these constituent elements, the slide search execution unit 1102a is a unit that performs various analyses for the target sequence by dividing the target sequence into partial sequences each having the user's designated length from the top of the target sequence, and searching the partial sequences from the sequence information on the population. The table generation unit 1102b is a unit that forms an analysis result of the slide search as well as the related information and the like into a table according to the output form set by the parameters and the like designated by the user in advance. Details of processings performed by these units will be explained later.

Fig. 13 is a block diagram of one example of a configuration of the slide search execution unit 1102a. Fig. 13 conceptually depicts only constituent elements related to the present invention among all constituent elements of the slide search execution unit 1102a. The slide search execution unit 1102a schematically includes a search condition setting unit 1102c, a simple slide search (SSS) execution unit 1102d, and a mutation slide search (MSS) execution unit 1102e.

Among the constituent elements, the search condition setting unit 1102c is a unit that causes the user to set various conditions for the slide search. The search condition setting unit 1102c functions as a target sequence selection unit that causes the user to select the target sequence among the sequence information stored in the in-house database 1106b, a population selection unit that causes the user to select the sequence information on the population, as the search target, among the sequence information stored in the protein set (PS) database 1106a, a unit that causes the user to designate the length of each partial sequence, a unit that causes the user to select whether to conduct a simple slide search or a mutation slide search, to be explained later, and the like.

The simple slide search (SSS) execution unit 1102d is a unit that executes the simple slide search to be explained later. The mutation slide search (MSS) execution unit 1102e is a unit that executes the mutation slide search, to be explained later, for searching the search target while enlarging the target range up to mutants of the partial sequences.

Fig. 14 is a block diagram of one example of a configuration of the simple slide search (SSS) execution unit 1102d. Fig. 14 conceptually depicts only constituent elements related to the present invention among all constituent elements of the simple slide search (SSS) execution unit 1102d. The simple slide search (SSS) execution unit 1102d schematically includes an oligopeptide sequence generation unit 1102k, a protein set database (PSDB) search unit 1102m, and an analysis result generation unit 1102n.

Among the constituent elements, the oligopeptide sequence generation unit 1102k is a sequence division unit that divides the target sequence into the partial sequences each having the user's designated length. The protein set database (PSDB) search unit 1102m is a search unit that searches the partial sequences divided by the oligopeptide sequence generation unit 1102k from the population extracted from the protein set (PS) database 1106a. The analysis result generation unit 1102n is an analysis result information generation unit that generates an analysis result about the target sequence based on a search result of the oligopeptide sequence generation unit 1102k.

Fig. 15 is a block diagram of one example of a configuration of the mutation slide search (MSS) execution unit 1102e, and conceptually depicts only constituent elements related to the present invention among all constituent elements of the mutation slide search (MSS) execution unit 1102e. The mutation slide search (MSS) execution unit 1102e schematically includes an oligopeptide sequence generation unit 1102p, a mutation table search unit 1102q, a protein set database (PSDB) search unit 1102r, and an analysis result generation unit 1102s.

Among the constituent elements, the oligopeptide sequence generation unit 1102p is a sequence division unit that divides the target sequence into the partial sequences each having the user's designated length. The mutation table search unit 1102q is a unit that acquires the mutants of the partial sequences from the mutation table 1106c. The protein set database (PSDB) search unit 1102r is a search unit that searches the partial sequences divided by the oligopeptide sequence generation unit 1102p from the population extracted from the protein set (PS) database 1106a. The analysis result generation unit 1102s is an analysis result information generation unit that generates an analysis result about the target sequence based on a search result of the protein set database (PSDB) search unit 1102r.

Fig. 16 is a block diagram of one example of a configuration of.the table generation unit 1102b. Fig. 16 conceptually depicts only constituent elements related to the present invention among all constituent elements of the table generation unit 1102b. The table generation unit 1102b schematically includes a parameter setting unit 1102f, a column data generation unit 1102g, an external analysis program execution unit 1102h, a table shaping unit 1102i, and an output form conversion unit 1102j.

Among the constituent elements, the parameter setting unit 1102f is a parameter setting unit that causes the user to set various table parameters to be output. The column data generation unit 1102g is a column data generation unit that generates column data on the table from partial sequence information on the partial sequences and analysis result information on the slide search according to the user's set parameters. The external program execution unit 1102h is an external program execution unit that executes the external program (e.g., the homology search or the motif search) based on the analysis result information, and that adds an execution result to the analysis result information.

The table shaping unit 1102i is a table shaping unit that shapes the partial sequence information and the analysis result information into a table according to the user's designated parameters. The output form conversion unit 1102j is an output form conversion unit that converts a form of the table data shaped by the table shaping unit 1102i into a user's designated display data description language (e.g., SGML, HTML, XML, or an original description language).

### [Processings Performed by System]

One example of the processings performed by the system thus constituted according to this embodiment will be explained in detail with reference to Figs. 17 to 23.

### [Slide Search Processing]

Details of a slide search processing will be explained with reference to flowcharts shown in Figs. 17 to 19 and display screen views and the like shown in Figs. 23 to 28. Fig. 17 is a flowchart of one example of the slide search processing performed by the system according to this embodiment.

The sequence information processing apparatus 100 causes the user to set various conditions for the slide search, display conditions for the table that displays the analysis result of the slide search and the like, etc. by the processing performed by the search condition setting unit 1102c (at a step SBB-1).

Fig. 23 depicts one example of a table parameter setting screen output to the output device 1114 by the processing performed by the search condition setting unit 1102c. As shown in Fig. 23, the table parameter setting screen includes an input area MAA-1 for inputting a table name, an input area MAA-2 for inputting a database name and a protein name of the target protein, an input area MAA-3 for inputting an oligopeptide length, an input area MAA-4 for inputting the number of header rows, an input area MAA-5 for inputting the number of columns of the table, and the like.

When the user completes inputting respective items of the MAA-1 to the MAA-5 on the parameter setting screen using the input device 1112, the search condition setting unit 1102c stores the input information in the storage unit 1106.

It is noted that the length of each partial sequence input to the oligopeptide length input area MAA-2 is preferably 2 to 10, more preferably, 4 to 7, most preferably 4 or 5.

Further, when the user inputs the number of header rows in the number-of-header-rows input area MAA-4, the search condition setting unit 1102c displays header parameter setting screen link buttons (corresponding to "header row 1" and "header row 2" in Fig. 23) as many as the input header rows as shown in Fig. 23. When the user selects one of the header parameter setting screen link buttons using the input device, the search condition setting unit 102c displays a header parameter setting screen shown in Fig. 27 on the output device 1114.

Fig. 27 depicts one example of the header parameter setting screen output to the output device 1114 by the processing performed by the search condition setting unit 1102c.

As shown in Fig. 27, the header parameter setting screen includes a display area MEE-1 for displaying a header row number and a total number of columns, an input area MEE-2 for inputting header format information composed by starting column numbers, end column numbers, and display items, and the like. When the user sets the end column numbers in the header format information input area MEE-2, the search condition setting unit 1102c automatically displays a next starting column number.

When the user completes inputting respective items of the header format information input area MEE-2 on the header parameter setting screen using the input device 1112, the search condition setting unit 1102c stores the input information in the storage unit 1106.

Referring back to Fig. 23, when the user inputs the number of columns of the table in the number-of-columns input area MAA-5, the search condition setting unit 1102c displays column parameter setting screen link buttons (corresponding to "column 1" to "column 14" shown in Fig. 23) as many as the columns as shown in Fig. 23. When the user selects one of the parameter setting screen link buttons using the input device 1112, the search condition setting unit 1102c displays a column parameter setting screen (1) shown in Fig. 24 on the output device 1114.

Fig. 24 depicts one example of the column parameter setting screen (1) output to the output device 1114 by the processing performed by the search condition setting unit 1102c.

As shown in Fig. 24, the column parameter setting screen (1) includes a selection button MBB-1 for selecting displaying of oligopeptides in the columns of the table; a selection button MBB-2 for selecting displaying of an execution result of the external analysis program in the columns of the table, an HTML link information input area MBB-3 for inputting an address and the like of the external analysis program, a selection button MBB-4 for selecting displaying of the analysis result information on the slide search in the columns of the table, and the like.

When the user selects the slide search result in the MBB-4, the search condition setting unit 1102c automatically displays a selection button MBB-5 for selecting a slide search type (SSS or MSS), an input area MBB-6 for inputting a name of the search target population, a selection area MBB-7 for selecting display information (e.g., the number of extracted proteins (for also selecting whether a display form is a number form or a graphic form (e.g., a graph)), a protein name, the number of extracted oligopeptides (for also selecting whether a display form is the number form or the graphic form (e.g., a graph), and a name of the protein in which oligopeptides are present and a position of the protein), a restriction parameter setting screen link button MBB-8 for moving a screen to a restriction parameter setting screen for setting restrictions when displaying information on the table, and the like.

When the user selects the mutation (MSS) on the selection button MBB-5 for selecting the slide search type (SSS or MSS), the search condition setting unit 1102c displays a column parameter setting screen (2) shown in Fig. 25.

Fig. 25 depicts one example of the column parameter setting screen (2) output to the output device 1114 by the processing performed by the search condition setting unit 1102c.

As shown in Fig. 25, the column parameter setting screen (2) includes a selection button MCC-1 for selecting displaying of oligopeptides in the columns of the table, a selection button MCC-2 for selecting displaying of the execution result of the external analysis program in the columns of the table, an HTML link information input area MCC-3 for inputting the address and the like of the external analysis program, a selection button MCC-4 for selecting displaying of the analysis result information on the slide search in the columns of the table, and the like.

When the user selects the slide search result on the MCC-4, the search condition setting unit 1102c automatically displays a selection button MCC-5 for selecting the slide search type (SSS or MSS), an input area MCC-6 for inputting the name of the search target population, a selection area MCC-7 for selecting display information (e.g., the number of extracted proteins (for also selecting whether the display form is the number form or the graphic form (e.g., a graph), whether a unit of accumulation is an oligopeptide unit or a mutant unit, and the like), a mutant name, the number of extracted oligopeptides (for also selecting whether the display form is the number form or the graphic form (e.g., a graph), whether the unit of accumulation is the oligopeptide unit or the mutant unit, and the like) and mutant sequences of oligopeptides, a name of the protein in which oligopeptide are present and positions of the proteins from the top of the amino acid sequence), a restriction parameter setting screen link button MCC-8 for moving a screen to a restriction parameter setting screen for setting restrictions when displaying information on the table, and the like.

When the user completes inputting and selecting the respective items on the column parameter setting screen (1) or the column parameter setting screen (2) using the input device 112, the search condition setting unit 1102c stores the input information in the storage unit 1106.

Further, when the user selects the restriction parameter setting screen link button MBB-8 or MCC-8 on the column parameter setting screen (1) or the column parameter setting screen (2), respectively, using the input device 112, the search condition setting unit 1102c displays a restriction parameter setting screen shown in Fig. 26.

Fig. 26 depicts one example of the restriction parameter setting screen output to the output device 1114 by the processing performed by the search condition setting unit 1102c. As shown in Fig. 26, the restriction parameter setting screen includes an input area MDD-1 for inputting an upper limit of the number of search results and an upper limit of the number of mutants, an input area MDD-2 for inputting an ignored oligopeptide in the target protein and a start position, an input area MDD-3 for inputting an ignored mutant, an ignored protein, and a protein and a start position, an input area MDD-4 for inputting the external analysis program, and the like.

When the user completes inputting the respective items of the MDD-1 to MDD-4 on the restriction parameter setting screen using the input device 1112, the search condition setting unit 1102c stores the input information in the storage unit 1106.

The slide search execution unit 1102a then accesses the in-house database 1106b, and acquires the user's designated target sequence (at a step SBB-2).

The slide search execution unit 1102a accesses the protein set (PS) database 1106a, extracts the sequence information coincident with the user's designated population conditions, and generates the population (at a step SBB-3).

The slide search execution unit 1102a determines the search type designated by the user (at a step SBB-4). If the search type is the SSS, the slide search execution unit 1102a executes an SSS processing, to be explained later, by the processing performed by the simple slide search (SSS) execution unit 1102d (at a step SBB-5).

If the search type is the MSS, the slide search execution unit 1102a executes an MSS processing, to be explained later, while referring to the mutation table 1106c by the processing performed by the mutation slide search (MSS) execution unit 1102e (at a step SBB-6).

The slide search execution unit 1102a stores analysis result information on the SSS or the MSS in the storage unit 1106 (at a step SBB-7). The slide search processing is thus finished.

### [Simple Slide Search (SSS) Processing]

Details of the simple slide search (SSS) processing will be explained with reference to Fig. 18. Fig. 18 is a flowchart of one example of the simple slide search (SSS) processing performed by the system according to this embodiment.

The simple slide search (SSS) execution unit 1102d first sets information (i) that indicates a position from the top of the target sequence at 1 (at a step SCC-1).

The oligopeptide sequence generation unit 1102k acquires the partial sequence (oligopeptide sequence) having the length set by the user in advance from a position i of the target sequence (amino acid sequence) (at a step SCC-2).

Fig. 21 is a conceptual view of one example of acquiring the partial sequence (oligopeptide sequence) having the length set by the user in advance from the position i of the target sequence (amino acid sequence). Fig. 22 depicts a list of three-letter symbols corresponding to respective one-letter symbols of amino acids used in Fig. 21 and names of the amino acids.

The protein set database (PSDB) search unit 1102m searches the oligopeptide sequence from the population' (at a step SCC-3). If the oligopeptide sequence is present in the population (at a step SCC-4), the protein set database (PSDB) search unit 1102m extracts the i, the oligopeptide sequence used in the search, the number of oligopeptides present in the population, and the name of the protein in which the oligopeptide sequence is present as search result information, and stores the search result information in the storage unit 1106 (at a step SCC-5).

The simple slide search (SSS) execution unit 1102d increments the i by 1 (at a step SCC-6).

The simple slide search (SSS) execution unit 1102d determines whether the i is at a last position of the target sequence (at a step SCC-7). If the i is not at the last position, the processing is returned to the step SCC-2.

If the i is at the last position of the target sequence, the analysis result generation unit 1102n accumulates search result information on all pieces of partial sequence information, and generates analysis result information on the target sequence (at a step SCC-8). The simple slide search (SSS) processing is thus finished.

### [Mutation Slide Search (MSS) Processing]

Details of a mutation slide search (MSS) processing will be explained with reference to Fig. 19. Fig. 19 is a flowchart of one example of the mutation slide search (MSS) processing performed by the system according to the embodiment.

The mutation slide search (MSS) execution unit 1102e first sets the information (i) that indicates the position from the top of the target sequence at 1 (at a step SDD-1).

The oligopeptide sequence generation unit 1102p acquires the oligopeptide sequence having the length set by the user in advance from the position i of the target sequence (amino acid sequence) (at a step SDD-2).

Fig. 21 is a conceptual view of one example of acquiring the partial sequence (oligopeptide sequence) having the length set by the user in advance from the position i of the target sequence (amino acid sequence). Fig. 22 depicts a list of three-letter symbols corresponding to respective one-letter symbols of amino acids used in Fig. 21 and names of the amino acids.

The mutation table search unit 1102q accesses the mutation table 1106c based on the oligopeptide sequence, and acquires the sequence of a mutant corresponding to the oligopeptide sequence (at a step SDD-3).

The protein set database (PSDB) search unit 1102r searches the oligopeptide sequence and its mutant from the population (at a step SDD-4). If the oligopeptide sequence or its mutant is present in the population (at a step SDD-5), the mutation table search unit 1102r extracts the i, the oligopeptide or mutant sequence used in the search, the number of oligopeptides present in the population, and the name of the protein in which the oligopeptide or mutant sequence is present as search result information, and stores the search result information in the storage unit 1106 (at a step SDD-6).

The mutation slide search (MSS) execution unit 1102e increments i by 1 (at a step SDD-7).

The mutation slide search (MSS) execution unit 1102e determines whether the i is at the last position of the target sequence (at a step SDD-8). If the i is not at the last position, the processing is returned to the step SDD-2.

If the i is at the last position of the target sequence, the analysis result generation unit 1102s accumulates search result information on all pieces of partial sequence information, and generates analysis result information on the target sequence (at a step SDD-9). The mutation slide search (MSS) processing is thus finished.

### [Table Generation Processing]

Details of a table generation processing will be explained with reference to Fig. 20. Fig. 20 is a flowchart of one example of the table generation processing performed by the system according to this embodiment.

The parameter setting unit 1102f first acquires various table parameters set by the user in advance while referring to the storage unit 1106 (at a step SEE-1). Namely, the parameter setting unit 1102f acquires the parameters set by the user using the various parameter setting screens and the like shown in Figs. 23 to 27, while referring to the storage unit 1106.

The table generation unit 1102b acquires the analysis result information on the slide search processing performed by the slide search execution unit 1102a, which information is stored in the storage unit 1106 (at a step SEE-2).

The column data generation unit 1102g generates table column data for the respective items of the analysis result information according to the table parameters set by the user. The column data generation unit 1102g determines whether calling of the external analysis program is designated for each column (at a step SEE-3).

If execution of the external analysis program is not selected at the step SEE-3, the column data generation unit 1102g generates column data based on the analysis result of the slide search (at a step SEE-4).

If execution of the external analysis program (e.g., the homology search or the motif search) is selected at the step SEE-3, the column data generation unit 1102g transmits a command, a factor, and the like to the external system 1200 designated by the HTML link information through the communication control interface unit 1104. In addition, the column data generation unit 1102g requests the external system 1200 to execute the external analysis program, and receives an execution result of the external analysis program from the external system 1200 (at a step SEE-5).

The column data generation unit 1102g generates column data based on the execution result of the external analysis program and the analysis result of the slide search (at a step SEE-6).

After generation of the column data and execution of the external analysis program are completed for all the columns, the table generation unit 1102b shapes the table for the execution result of the external analysis program and the like so that the user can easily see the table and for the number of columns and the number of rows of the table to be displayed by the processing performed by the table shaping unit 1102i (at a step SEE-7). In addition, the table generation unit 1102b converts a form of the table data into a table display data description language (e.g., HTML or XML) set by the user using a known conversion technique by the processing performed by the output form conversion unit 1102j (at a step SEE-8).

The output form conversion unit 1102j stores the generated table display data in the storage unit 1106 (at a step SEE-9), and transmits and outputs the data to the output device 1114 at a desired timing.

Fig. 28 depicts one example of a table output to the output device 1114 of the sequence information processing apparatus 1100. As shown in Fig. 28, the table includes an output area MFF-1 for outputting a header and the column data corresponding to the header.

In the example of Fig. 28, the header includes a position from the top MFF-2, an oligopeptide sequence MFF-3 that is the partial sequence of the target sequence, the number of extracted sequences MFF-4 from the population and graphic display of the number, a mutant sequence MFF-5, a name of each of the extracted proteins MFF-6, a population extraction condition MFF-7, and a search type MFF-8.

Further, WWW link information on a related website is buried in each protein name. By user's clicking on the protein name, information on the website can be displayed using a technique for displaying a webpage having a known WWW hyperlink structure (MFF-9).

If the number of extracted oligopeptides exceeds an upper limit of the user's set number of extracted oligopeptides, it is possible to exercise a control so as not to display information in vast amounts (MFF-10). The table generation processing is thus finished.

### [Other embodiments]

While exemplary embodiments of the present invention have been explained above, the invention may be also practiced by various embodiments other than the above-described ones, within the technical scope of the appended claims.

For example, the various pieces of information input by the user on the table parameter setting screen, the column parameter setting screen, the header parameter setting screen, the restriction parameter setting screen, and the like may be defined and set by designating the data in a program form in a script language or the like.

Of the respective processing explained in the embodiments, all or a part of the processing explained as being performed automatically may be performed manually, or all or a part of the processing explained as being performed manually may be performed automatically in a known method.

In addition, information that includes the processing procedure, the control procedure, specific names, various kinds of registered data, and parameters such as search conditions, shown in the specification or in the drawings, as well as screen examples and database configurations can be optionally changed, unless otherwise specified.

As for the sequence information processing apparatus 1100, the respective constituent elements of the apparatus 1100 shown in the drawings are functionally conceptual and are not necessarily constituted physical as shown in the drawings.

For example, all or an arbitrary part of the processing functions of the respective units or devices included in the sequence information processing apparatus 1100 can be realized by the CPU (central processing unit) and a program interpreted and executed by the CPU, or can be realized as wired logic-based hardware. The program is recorded in a recording medium to be explained later, and mechanically read by the sequence information processing apparatus 1100 if necessary.

The various databases and the like (the protein set (PS) database 1106a to the table 1106d) stored in the storage unit 1106 are storage units such as memory devices, e.g., the RAM and the ROM, fixed disk devices such as hard disks, flexible disks, and optical disks. These databases and the like store various programs, tables, files, databases, webpage files, and the like used for various processings and for providing websites, respectively.

The sequence information processing apparatus 1100 may be realized by connecting peripherals such as a printer, a monitor, and an image scanner to an information processing apparatus such as a known information processing terminal, e.g., a personal computer or a workstation, and installing software (including the program, the data, and the like) for realizing the method according to the present invention into the information processing apparatus.

Furthermore, specific forms of distribution or integration of the sequence information processing apparatus 1100 are not limited to those shown in the drawings. The apparatus 1100 can be constituted by distributing or integrating all or a part of the apparatus 1100 either functionally or physically in arbitrary units according to various loads and the like. For example, the respective databases may be constituted independently as independent database devices, or part of the processings may be realized by the CGI (common gateway interface).

The program according to the present invention can be stored in a computer readable recording medium. It is assumed herein that the "recording medium" includes arbitrary "portable physical medium" such as a flexible disk, a magneto-optical disk, a ROM, an EPROM, an EEPROM, a CD-ROM, an MO, and a DVD, arbitrary "fixed physical medium" such as a ROM, a RAM, and an HD included in various computer systems, and "communication medium" that temporarily holds the program such as a communication line or a carrier wave used when the program is transmitted through the network represented by a LAN, a WAN, or the internet.

Furthermore, the "program" signifies a data processing method described in an arbitrary language or by an arbitrary description method, and a form of the program is not limited to a source code, a binary code, or the like. It is noted that the "program" is not always limited to the program constituted singularly. Examples of the "program" include a program that is distribution-structured as a plurality of modules or libraries, or attains its functions in cooperation with another program represented by the OS (Operating System). As for specific configurations and reading procedures for reading the recording medium by the respective devices explained in the embodiment as well as installation procedures and the like after reading, well-known configurations and procedures can be also used.

Additionally, the network 1300 functions to connect the mutant sequence analyzing apparatus 1100 to the external system 1200. The network 1300 may include, for example, any one of the Internet, an Intranet, a LAN (which may be wired or wireless), a VAN, a personal computer communication network, a public telephone network (which may be analog or digital), a CATV network, a portable line exchange network and portable packet exchange network of an IMT2000, a GSM, or PDC/PDC-P, a wireless call network, a local wireless network such as Bluetooth, a PHS network, and satellite communication networks such as CS, BS, and ISDB. Namely, the system according to the present invention can transmit and receive various pieces of data through the arbitrary network whether the network is wired or wireless.

As explained so far in detail, according to the present invention, sequence information on bases or amino acids and sequence related information related to the sequence information is stored white making the sequence information and the sequence related information correspond to each other, a user is caused to select a target sequence from among the stored sequence information, the user is caused to select sequence information on a population, which is a search target, from among the stored sequence information, the selected target sequence is divided into partial sequences each having a length designated by the user, the divided partial sequences are searched from the population, analysis result information on the target sequence is generated from search result information, and partial sequence information on the partial sequences and the analysis result information is output in a table form according to parameters set by the user. Therefore, it is possible to provide the sequence information processing apparatus, the sequence information processing method, the program, and the recording medium capable of allowing the user to easily make a setting as to which sequence information and which analysis information on the sequence information is to be chosen to be displayed as a processing result, to store the setting, and to reuse the setting later.

According to the present invention, the sequence information and mutant sequence information on mutant sequences which may possibly be generated by mutation of the sequence information is stored while making the sequence information and the mutant sequence information correspond to each other, and mutant sequence information on the divided partial sequences is searched from the stored mutant sequence information, and the mutant information on the searched partial sequences searched is searched from the population. Therefore, it is possible to provide the sequence information processing apparatus, the sequence information processing method, the program, and the recording medium capable of enlarging a search range up to mutants of the search target partial sequences, and using the information to analyze more useful biological information.

According to the present invention, the sequence related information includes information on at least one of a name of each sequence, a name of an organism from which the each sequence is derived, a name of a corresponding protein, functions of the protein, and an address of a related database. Therefore, it is possible to provide the sequence information processing apparatus, the sequence information processing method, the program, and the recording medium capable of using these items of information as analysis information on the target sequence.

According to the present invention, the population is generated by collecting sequence information on proteins that possess specific properties. Therefore, it is possible to provide the sequence information processing apparatus, the sequence information processing method, the program, and the recording medium capable of limiting the proteins to the proteins necessary to extract as the analysis information, and reducing search time. In addition, it is possible to provide the sequence information processing apparatus, the sequence information processing method, the program, and the recording medium capable of analyzing the population with respect to another target sequence.

According to the present invention, the length of each of the partial sequences is 4 to 7. Therefore, it is possible to provide the sequence information processing apparatus, the sequence information processing method, the program, and the recording medium capable of accumulating biologically effective data within operation time of an appropriate computer.

According to the present invention, the partial sequence information includes information on at least one of a position from a top of the target sequence and the partial sequences. Therefore, it is possible to provide the sequence information processing apparatus, the sequence information processing method, the program, and the recording medium capable of arbitrarily displaying each partial sequence and its position.

According to the present invention, the analysis result information includes information on at least one of the number of extracted sequences, the mutant sequence information used in a search, a name of each of the extracted sequences, a name of an organism from which the each extracted sequence is derived, a name of a protein corresponding to the each extracted sequence, a function of the protein corresponding to the each extracted sequence, an execution result of an external program, and an address of a related database to the each extracted sequence, for each of the partial sequences. Therefore, it is possible to provide the sequence information processing apparatus, the sequence information processing method, the program, and the recording medium capable of arbitrarily displaying these respective items of information.

Furthermore, according to the present invention, the parameters include information on at least one of whether each information included in the analysis result information can be output, an upper limit of the number of output sequences among the extracted sequences, whether the each information included in the analysis result information can be graphically displayed, a header format of the table, and a data description language for displaying data in the table. Therefore, it is possible to provide the sequence information processing apparatus, the sequence information processing method, the program, and the recording medium capable of arbitrarily displaying these respective pieces of information.

### INDUSTRIAL APPLICABILITY

(I) As explained so far, the mutant sequence analyzing apparatus, the mutant sequence analyzing method, the program, and the recording medium can efficiently check a mutant status and the like of a DNA sequence or the like of a mutant at real time using sequence data obtained from a sequencing experiment.

Namely, the mutant sequence analyzing apparatus, the mutant sequence analyzing method, the program, and the recording medium according to the present invention can efficiently execute assembling of the DNA sequence of the mutant and identification of the mutant part.

Accordingly, the mutant sequence analyzing apparatus, the mutant sequence analyzing method, the program, and the recording medium according to the present invention are remarkably useful in the bioinformatics field of analyzing DNA sequences and the like.

Since the present invention can be thereby carried out widely in many industrial fields, particularly fields of pharmaceuticals, foods, cosmetics, medial treatment, gene expression analysis, and the like, the invention is remarkably useful.

(II) In addition, as explained so far, the sequence information processing apparatus, the sequence information processing method, the program, and the recording medium enable the user to easily make a setting as to which sequence information and which related information to the sequence information is to be chosen so as to display the information on the output device, to store the setting, and to search the setting later so that the user can reuse the setting.

The sequence information processing apparatus, the sequence information processing method, the program, and the recording medium are, therefore, remarkably useful in the bioinformatics field of analyzing DNA sequences, amino acid sequences, and the like.

Since the present invention can be thereby carried out widely in many industrial fields, particularly fields of pharmaceuticals, foods, cosmetics, medial treatment, gene expression analysis, and the like, the invention is remarkably useful.

## Claims

1. A mutant sequence analyzing apparatus comprising:
a fragment sequence storage unit that stores fragment sequence information on a base sequence of each DNA fragment of a mutant;
a genome information storage unit that stores original DNA sequence information before mutation;
a region-of-interest selection unit that selects a region of interest composed by a sequence region of at least a part of the original DNA sequence information stored in the genome information storage unit;
a homology search unit that determines a similarity between the region of interest selected by the region-of-interest selection unit and the fragment sequence information stored in the fragment sequence storage unit;
a fragment adoption determination unit that determines whether to adopt the fragment sequence information as the fragment sequence information used for sequencing the mutation, according to the similarity determined by the homology search unit;
an alignment processing unit that makes an alignment between the fragment sequence information determined to be adopted by the fragment adoption determination unit and the region of interest; and
a consensus sequence determination unit that determines a consensus sequence of the mutant based on an alignment result of the alignment processing unit.

2. The mutant sequence analyzing apparatus according to claim 1, further comprising a mutation information determination unit that determines mutation information by comparing the consensus sequence determined by the consensus sequence determination unit with the region of interest.

3. The mutant sequence analyzing apparatus according to claim 1, further comprising a redundancy determination unit that determines a redundancy of the each fragment relative to each base that constitutes the region of interest based on the alignment result of the alignment processing unit.

4. The mutant sequence analyzing apparatus according to claim 1, further comprising a gap information determination unit that determines gap information that specifies a gap part of the each fragment relative to the region of interest based on the alignment result of the alignment processing unit.

5. The mutant sequence analyzing apparatus according to claim 1, further comprising a processing result display unit that displays a processing result including at least one of the region of interest, annotation information on the region of interest, the consensus sequence, annotation information on the consensus sequence, the redundancy, the mutation information, the gap information, and the alignment result.

6. The mutant sequence analyzing apparatus according to claim 5, wherein
the annotation information on the consensus sequence includes information on at least one of an ORF region of the consensus sequence, a control region, an amino acid sequence after translation, an original amino acid sequence before mutation, a function after the mutation, an original function before the mutation, and an orthologue relationship.

7. The mutant sequence analyzing apparatus according to claim 5, wherein
the processing result display unit displays the processing result on a plurality of output devices by dividing the processing result by as much as the number of the output devices.

8. The mutant sequence analyzing apparatus according to claim 1, further comprising a primer sequence determination unit that determines base sequence candidates of a PCR primer used to generate the each fragment that includes both of or one of a sequence region having both of or one of the redundancy determined by the redundancy determination unit and a sequence quality, each of which is lower than a preset value, and a sequence region of the gap part determined by the gap information determination unit.

9. The mutant sequence analyzing apparatus according to claim 8, wherein
the primer sequence determination unit further comprises:
a partial sequence extraction unit that extracts partial sequences located outside of both of or one of the sequence region having both of or one of the redundancy determined by the redundancy determination unit and the sequence quality, each of which is lower than the preset value, and the sequence region of the gap part determined by the gap information determination unit, and each having a high specificity in each of the sequences; and
a primer candidate selection unit that determines the base sequence candidates of the PCR primer among the partial sequences extracted by the partial sequence extraction unit according to preset selection conditions.

10. The mutant sequence analyzing apparatus according to claim 9, wherein
each of the partial sequences is a base sequence having the number of bases equal to or greater than 15 and equal to or smaller than 30.

11. A mutant sequence analyzing method comprising:
a fragment sequence storage step of storing fragment sequence information on a base sequence of each DNA fragment of a mutant;
a genome information storage step of storing original DNA sequence information before mutation;
a region-of-interest selection step of selecting a region of interest composed by a sequence region of at least a part of the original DNA sequence information stored at the genome information storage step;
a homology search step of determining a similarity between the region of interest selected at the region-of-interest selection step and the fragment sequence information stored at the fragment sequence storage step;
a fragment adoption determination step of determining whether to adopt the fragment sequence information as the fragment sequence information used for sequencing the mutation, according to the similarity determined at the homology search step;
an alignment processing step of making an alignment between the fragment sequence information determined to be adopted at the fragment adoption determination step and the region of interest; and
a consensus sequence determination step of determining a consensus sequence of the mutant based on an alignment result of the alignment processing step.

12. The mutant sequence analyzing method according to claim 11, further comprising a mutation information determination step of determining mutation information by comparing the consensus sequence determined at the consensus sequence determination step with the region of interest.

13. The mutant sequence analyzing method according to claim 11, further comprising a redundancy determination step of determining a redundancy of the each fragment relative to each base that constitutes the region of interest based on the alignment result of the alignment processing step.

14. The mutant sequence analyzing method according to claim 11, further comprising a gap information determination step of determining gap information that specifies a gap part of the each fragment relative to the region of interest based on the alignment result of the alignment processing step.

15. The mutant sequence analyzing method according to claim 11, further comprising a processing result display step of displaying a processing result including at least one of the region of interest, annotation information on the region of interest, the consensus sequence, annotation information on the consensus sequence, the redundancy, the mutation information, the gap information, and the alignment result.

16. The mutant sequence analyzing method according to claim 15, wherein
the annotation information on the consensus sequence includes information on at least one of an ORF region of the consensus sequence, a control region, an amino acid sequence after translation, an original amino acid sequence before mutation, a function after the mutation, an original function before the mutation, and an orthologue relationship.

17. The mutant sequence analyzing method according to claim 15, wherein
at the processing result display step, the processing result is displayed for a plurality of output methods by dividing the processing result by as much as the number of the output methods.

18. The mutant sequence analyzing method according to claim 11, further comprising a primer sequence determination step of determining base sequence candidates of a PCR primer used to generate the each fragment that includes both of or one of a sequence region having both of or one of the redundancy determined at the redundancy determination step and a sequence quality, each of which is lower than a preset value, and a sequence region of the gap part determined at the gap information determination step.

19. The mutant sequence analyzing method according to claim 18, wherein
the primer sequence determination step further comprises:
a partial sequence extraction step of extracting partial sequences located outside of both of or one of the sequence region having both of or one of the redundancy determined at the redundancy determination step and the sequence quality, each of which is lower than the preset value, and the sequence region of the gap part determined at the gap information determination step, and each having a high specificity in each of the sequences; and
a primer candidate selection step of determining the base sequence candidates of the PCR primer among the partial sequences extracted at the partial sequence extraction step according to preset selection conditions.

20. The mutant sequence analyzing method according to claim 19, wherein
each of the partial sequences is a base sequence having the number of bases equal to or greater than 15 and equal to or smaller than 30.

21. A program that makes a computer execute a mutant sequence analyzing method comprising:
a fragment sequence storage step of storing fragment sequence information on a base sequence of each DNA fragment of a mutant;
a genome information storage step of storing original DNA sequence information before mutation;
a region-of-interest selection step of selecting a region of interest composed by a sequence region of at least a part of the original DNA sequence information stored at the genome information storage step;
a homology search step of determining a similarity between the region of interest selected at the region-of-interest selection step and the fragment sequence information stored at the fragment sequence storage step;
a fragment adoption determination step of determining whether to adopt the fragment sequence information as the fragment sequence information used for sequencing the mutation, according to the similarity determined at the homology search step;
an alignment processing step of making an alignment between the fragment sequence information determined to be adopted at the fragment adoption determination step and the region of interest; and
a consensus sequence determination step of determining a consensus sequence of the mutant based on the alignment result of the alignment processing step.

22. The program according to claim 21, further comprising a mutation information determination step of determining mutation information by comparing the consensus sequence determined at the consensus sequence determination step with the region of interest.

23. The program according to claim 21, further comprising a redundancy determination step of determining a redundancy of the each fragment relative to each base that constitutes the region of interest based on the alignment result of the alignment processing step.

24. The program according to claim 21, further comprising a gap information determination step of determining gap information that specifies a gap part of the each fragment relative to the region of interest based on the alignment result of the alignment processing step.

25. The program according to claim 21, further comprising a processing result display step of displaying a processing result including at least one of the region of interest, annotation information on the region of interest, the consensus sequence, annotation information on the consensus sequence, the redundancy, the mutation information, the gap information, and the alignment result.

26. The program according to claim 25, wherein
the annotation information on the consensus sequence includes information on at least one of an ORF region of the consensus sequence, a control region, an amino acid sequence after translation, an original amino acid sequence before mutation, a function after the mutation, an original function before the mutation, and an orthologue relationship.

27. The program according to claim 25, wherein
at the processing result display step, the processing result is displayed for a plurality of output methods by dividing the processing result by as much as the number of the output methods.

28. The program according to claim 21, further comprising a primer sequence determination step of determining base sequence candidates of a PCR primer used to generate the each fragment that includes both of or one of a sequence region having both of or one of the redundancy determined at the redundancy determination step and a sequence quality, each of which is lower than a preset value, and a sequence region of the gap part determined at the gap information determination step.

29. The program according to claim 28, wherein
the primer sequence determination step further comprises:
a partial sequence extraction step of extracting partial sequences located outside of both of or one of the sequence region having both of or one of the redundancy determined at the redundancy determination step and the sequence quality, each of which is lower than the preset value, and the sequence region of the gap part determined at the gap information determination step, and each having a high specificity in each of the sequences; and
a primer candidate selection step of determining the base sequence candidates of the PCR primer among the partial sequences extracted at the partial sequence extraction step according to preset selection conditions.

30. The program according to claim 29, wherein
each of the partial sequences is a base sequence having the number of bases equal to or greater than 15 and equal to or smaller than 30.

31. A computer readable recording medium having the program according to any one of claims 21 to 30 recorded therein.

32. A sequence information processing apparatus comprising:
a sequence information storage unit that stores sequence information on bases or amino acids and sequence related information related to the sequence information while making the sequence information and the sequence related information correspond to each other;
a target sequence selection unit that causes a user to select a target sequence from among the sequence information stored in the sequence information storage unit;
a population selection unit that causes the user to select sequence information on a population, which is a search target, from among the sequence information stored in the sequence information storage unit;
a sequence division unit that divides the target sequence selected by the target sequence selection unit into partial sequences each having a length designated by the user;
a search unit that searches the partial sequences divided by the sequence division unit from the population;
an analysis result information generation unit that generates analysis result information on the target sequence from search result information on the search unit; and
a table output unit that outputs partial sequence information on the partial sequences and the analysis result information in a table form according to parameters set by the user.

33. The sequence information processing apparatus according to claim 32, further comprising:
a mutant sequence information storage unit that stores the sequence information and mutant sequence information on mutant sequences which may possibly be generated by mutation of the sequence information while making the sequence information and the mutant sequence information correspond to each other; and
a mutant sequence search unit that searches mutant sequence information on the partial sequences divided by the partial sequence division unit from the mutant sequence information stored in the mutant sequence information storage unit, wherein
the search unit searches the mutant information on the partial sequences searched by the mutant sequence search unit, from the population.

34. The sequence information processing apparatus according to claim 32, wherein
the sequence related information includes information on at least one of a name of the each sequence, a name of an organism from which the each sequence is derived, a name of a corresponding protein, functions of the protein, and an address of a related database.

35. The sequence information processing apparatus according to claim 32, wherein
the population is generated by collecting sequence information on proteins that possess specific properties.

36. The sequence information processing apparatus according to claim 32, wherein
the length of each of the partial sequences is 4 to 7.

37. The sequence information processing apparatus according to claim 32, wherein
the partial sequence information includes information on at least one of a position from a top of the target sequence and the partial sequences.

38. The sequence information processing apparatus according to claim 32, wherein
the analysis result information includes information on at least one of the number of extracted sequences, the mutant sequence information used in a search, a name of each of the extracted sequences, a name of an organism from which the each extracted sequence is derived, a name of a protein corresponding to the each extracted sequence, a function of the protein corresponding to the each extracted sequence, an execution result of an external program, and an address of a related database to the each extracted sequence, for each of the partial sequences.

39. The sequence information processing apparatus according to claim 32, wherein
the parameters include information on at least one of whether each information included in the analysis result information can be output, an upper limit of the number of output sequences among the extracted sequences, whether the each information included in the analysis result information can be graphically displayed, a header format of the table, and a data description language for displaying data in the table.

40. A sequence information processing method comprising:
a sequence information storage step of storing sequence information on bases or amino acids and sequence related information related to the sequence information while making the sequence information and the sequence related information correspond to each other;
a target sequence selection step of causing a user to select a target sequence from among the sequence information stored at the sequence information storage step;
a population selection step of causing the user to select sequence information on a population, which is a search target, from among the sequence information stored at the sequence information storage step;
a sequence division step of dividing the target sequence selected at the target sequence selection step into partial sequences each having a length designated by the user;
a search step of searching the partial sequences divided at the sequence division step from the population;
an analysis result information generation step of generating analysis result information on the target sequence from search result information on the search step; and
a table output step of outputting partial sequence information on the partial sequences and the analysis result information in a table form according to parameters set by the user.

41. The sequence information processing method according to claim 40, further comprising:
a mutant sequence information storage step of storing the sequence information and mutant sequence information on mutant sequences which may possibly be generated by mutation of the sequence information while making the sequence information and the mutant sequence information correspond to each other; and
a mutant sequence search step of searching mutant sequence information on the partial sequences divided at the partial sequence division step from the mutant sequence information stored at the mutant sequence information storage step, wherein
at the search step, the mutant information on the partial sequences searched by the mutant sequence search step is searched from the population.

42. The sequence information processing method according to claim 40, wherein
the sequence related information includes information on at least one of a name of each sequence, a name of an organism from which the each sequence is derived, a name of a corresponding protein, functions of the protein, and an address of a related database.

43. The sequence information processing method according to claim 40, wherein
the population is generated by collecting sequence information on proteins that possess specific properties.

44. The sequence information processing method according to claim 40, wherein
the length of each of the partial sequences is 4 to 7.

45. The sequence information processing method according to claim 40, wherein
the partial sequence information includes information on at least one of a position from a top of the target sequence and the partial sequences.

46. The sequence information processing method according to claim 40, wherein
the analysis result information includes information on at least one of the number of extracted sequences, the mutant sequence information used in a search, a name of each of the extracted sequences, a name of an organism from which the each extracted sequence is derived, a name of a protein corresponding to the each extracted sequence, a function of the protein corresponding to the each extracted sequence, an execution result of an external program, and an address of a related database to the each extracted sequence, for each of the partial sequences.

47. The sequence information processing method according to claim 40, wherein
the parameters include information on at least one of whether each information included in the analysis result information can be output, an upper limit of the number of output sequences among the extracted sequences, whether the each information included in the analysis result information can be graphically displayed, a header format of the table, and a data description language for displaying data in the table.

48. A program that makes a computer execute a sequence information processing method comprising:
a sequence information storage step of storing sequence information on bases or amino acids and sequence related information related to the sequence information while making the sequence information and the sequence related information correspond to each other;
a target sequence selection step of causing a user to select a target sequence from among the sequence information stored at the sequence information storage step;
a population selection step of causing the user to select sequence information on a population, which is a search target, from among the sequence information stored at the sequence information storage step;
a sequence division step of dividing the target sequence selected at the target sequence selection step into partial sequences each having a length designated by the user;
a search step of searching the partial sequences divided at the sequence division step from the population;
an analysis result information generation step of generating analysis result information on the target sequence from search result information on the search step; and
a table output step of outputting partial sequence information on the partial sequences and the analysis result information in a table form according to parameters set by the user.

49. The program according to claim 48, further comprising:
a mutant sequence information storage step of storing the sequence information and mutant sequence information on mutant sequences which may possibly be generated by mutation of the sequence information while making the sequence information and the mutant sequence information correspond to each other; and
a mutant sequence search step of searching mutant sequence information on the partial sequences divided at the partial sequence division step from the mutant sequence information stored at the mutant sequence information storage step, wherein
at the search step, the mutant information on the partial sequences searched by the mutant sequence search step is searched from the population.

50. The program according to claim 48, wherein
the sequence related information includes information on at least one of a name of each sequence, a name of an organism from which the each sequence is derived, a name of a corresponding protein, functions of the protein, and an address of a related database.

51. The program according to claim 48, wherein
the population is generated by collecting sequence information on proteins that possess specific properties.

52. The program according to claim 48, wherein
the length of each of the partial sequences is 4 to 7.

53. The program according to claim 48, wherein
the partial sequence information includes information on at least one of a position from a top of the target sequence and the partial sequences.

54. The program according to claim 48, wherein the analysis result information includes information on at least one of the number of extracted sequences, the mutant sequence information used in a search, a name of each of the extracted sequences, a name of an organism from which the each extracted sequence is derived, a name of a protein corresponding to the each extracted sequence, a function of the protein corresponding to the each extracted sequence, an execution result of an external program, and an address of a related database to the each extracted sequence, for each of the partial sequences.

55. The program according to claim 48, wherein
the parameters include information on at least one of whether each information included in the analysis result information can be output, an upper limit of the number of output sequences among the extracted sequences, whether the each information included in the analysis result information can be graphically displayed, a header format of the table, and a data description language for displaying data in the table.

56. A computer readable recording medium having the program according to any one of claims 48 to 55 recorded therein.
